# EUROPÄISCHE PATENTSCHRIFT

(11) **EP 0 704 444 B1**
(45) Veröffentlichungstag und Bekanntmachung des Hinweises auf die Patenterteilung: **25.10.2000**
(21) Anmeldenummer: 95810552.0
(22) Anmeldetag: 06.09.1995
(51) Int. Cl.: C07D 403/12, C08K 5/3492

(54) **UV-Absorber, ihre Herstellung und Verwendung**
Triazine UV-absorbers, their production and use
Triazines substitués, procédé pour leur préparation et leur utilasition comme absorbant d'UV

(30) Priorität: 14.09.1994 CH 280294
(43) Veröffentlichungstag der Anmeldung: 03.04.1996
(73) Patentinhaber: Ciba Specialty Chemicals Holding Inc., 4057 Basel (CH)
(72) Erfinder: Fuso, Francesco, Dr., CH-4106 Therwil (CH); Reinert, Gerhard, Dr., CH-4123 Allschwil (CH)

(56) Entgegenhaltungen:
- EP-A- 0 388 356
- EP-A- 0 510 448
- EP-A- 0 618 205
- DE-A- 1 965 585
- U. ZORLL: 'Römpp Lexikon :Lacke und Druckfarben', GEORG THIEME VERLAG, STUTTGART Seiten 295-297 und 593

## Beschreibung

Gegenstand der vorliegenden Erfindung sind neue faserreaktive UV-Absorber, Verfahren zu ihrer Herstellung und ihre Verwendung zur photochemischen Stabilisierung von ungefärbten und gefärbten Textilfasern und zur Verbesserung des Sonnenschutzfaktors von solchen Textilfasern.

Die hautschädigende Wirkung von UV-Strahlung ist bekannt. Üblicherweise schützt man sich vor starker Sonnenbestrahlung, indem man eine UV-Absorber enthaltende Zusammensetzung (Sonnencreme) direkt auf die Haut aufgeträgt. In besonders sonnigen Bereichen der Erde, z.B. in Australien oder Amerika, steigt jedoch die Rate der durch UV-Strahlung bedingten Hautschädigungen in neuerer Zeit drastisch an. Dementsprechend wird dem Schutz der Haut vor Sonnenbestrahlung in diesen Ländern eine erhöhte Aufmerksamkeit geschenkt.

Es ist daher vorgeschlagen worden, nicht nur die Haut direkt, sondern auch die sie umgebende Bekleidung sowie textile Sonnenschutzartikel wie Markisen oder Sonnenschirme zusätzlich gegen UV-Strahlung zu schützen. Die meisten natürlichen und synthetischen Textilmaterialien, ob ungefärbt oder gefärbt, sind nämlich zumindest teilweise durchlässig für UV-Strahlung, sodass das blosse Tragen von Bekleidung keinen angemessenen Schutz der Haut vor Schädigungen durch UV-Strahlung bietet. Abhilfe ist hier möglich durch die Einarbeitung von UV-Absorbern in das Textilgewebe.

Aus EP-A-0 618 205 sind Monotriazinverbindungen enthaltend sterisch gehinderte Amine und ihre Verwendung zur photochemischen und thermischen Stabilisierung von Polyamidfasermaterialien bekannt.
Die im Bereich der Textilmaterialien, insbesondere der Cellulosefasern oder natürliche oder synthetische Polyamidfasern enthaltenden Textilmaterialien, erzielten Ergebnisse bezüglich Schutz vor UV-Strahlung sind jedoch bisher noch nicht befriedigend, und es besteht ein Bedürfnis, neue speziell auf diese Materialien abgestimmte UV-Absorber zu entwickeln.

Gegenstand der vorliegenden Erfindung sind daher Verbindungen der Formel 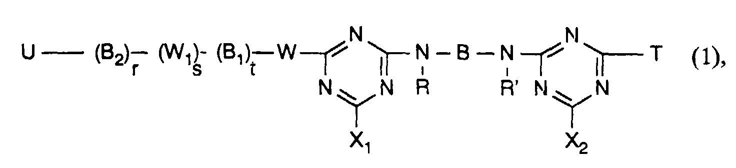 worin
B ein geradkettiges oder verzweigtes und gegebenenfalls durch Hydroxy, Sulfo oder Sulfato substituiertes und/oder durch -O- unterbrochenes C₂-C₁₂-Alkylen, unsubstituiertes oder durch Sulfo, Carboxy, C₁-C₄-Alkyl, C₁-C₄-Alkoxy oder Halogen substituiertes 1,2-, 1,3- oder 1,4-Phenylen, gegebenenfalls durch Sulfo substituiertes Naphthylen, Rest der Formel 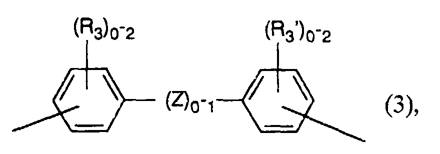 worin Z die Bedeutung -CO-, -NHCO-, -NHCONH-, -(CH₂)₁₋₄-, -NH-, -CH=CH-, -O-, -SO₂- oder -N=N- hat und (R₃)₀₋₂ und (R₃')₀₋₂ unabhängig voneinander jeweils für 0 bis 2 gleiche oder verschiedene Reste aus der Gruppe Sulfo, Methyl, Methoxy und Chlor stehen, unsubstituiertes oder im Phenylenteil durch Sulfo, Methyl,Methoxy, Carboxy oder Chlor substituiertes Phenyl-C₁-C₄-Alkylen, Cyclohexylen, Rest der Formel 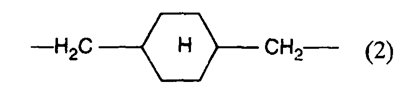 ist, oder zusammen mit -NR- und -NR'- einen heterocyclischen Ring bildet,
B₁ und B₂ unabhängig voneinander je ein geradkettiges oder verzweigtes C₁-C₁₂-Alkylen bedeuten,
R, R' und R₂ unabhängig voneinander je Wasserstoff oder gegebenenfalls durch Halogen, Hydroxy, Cyano, C₁-C₄-Alkoxy C₁-C₄-Alkoxycarbonyl, Carboxy, Sulfamoyl, Sulfo oder Sulfato substituiertes C₁-C₄-Alkyl bedeuten,
U der Rest eines 2-(2'-Hydroxyphenyl)-benztriazol-UV-Absorbers der Formel 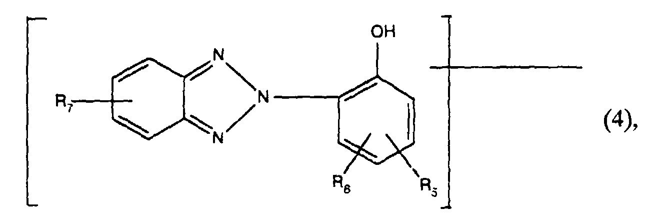 worin
R₅ Wasserstoff, C₁-C₁₂-Alkyl, Phenyl-C₁-C₄-alkyl, C₅-C₈-Cycloalkyl oder ein Rest der Formel 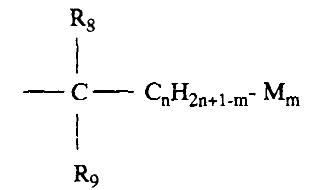 ist, worin R₈ und R₉
unabhängig voneinander Alkyl mit je 1 bis 5 Kohlenstoffatomen sind, oder Rg zusammen mit dem Rest CₙH₂ₙ₊₁₋ₘ einen Cycloalkylrest mit 5 bis 12 Kohlenstoffatomen bildet, m 1 oder 2, n eine ganze Zahl von 2 bis 20 und M ein Rest der Formel -COOR₁₀ ist, worin R₁₀ Wasserstoff, Alkyl mit 1 bis 12 Kohlenstoffatomen, Alkoxyalkyl mit je 1 bis 20 Kohlenstoffatomen im Alkyl- und Alkoxyteil oder Phenylalkyl mit 1 bis 4 Kohlenstoffatomen im Alkylteil ist,
R₆ Wasserstoff, Sulfo, Halogen, Alkyl mit 1 bis 18 Kohlenstoffatomen, Phenylalkyl mit 1 bis 4 Kohlenstoffatomen im Alkylteil, und
R₇ Wasserstoff, Sulfo, Chlor, Alkyl oder Alkoxy mit je 1 bis 4 Kohlenstoffatomen oder -COOR₁₀, worin R₁₀ die angegebene Bedeutung hat, bedeutet, wobei mindestens einer der Reste R₅ und R₆ von Wasserstoff verschieden ist,
eines 2-Hydroxybenzophenon-UV-Absorbers der Formel, 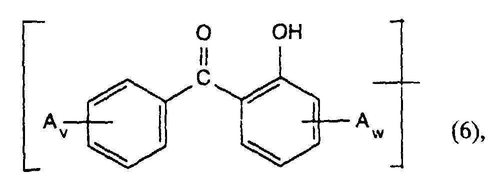 worin v eine ganze Zahl von 1 bis 3 und w 1 oder 2 ist, und die Substituenten A unabhängig voneinander Wasserstoff, Halogen, Hydroxyl, Sulfo, Alkoxy mit 1 bis 12 Kohlenstoffatomen oder Phenyl-C₁-C₄-Alkoxy sind,
eines 2-Hydroxyphenyl,1,3,5-triazin-UV-Absorbers der Formel 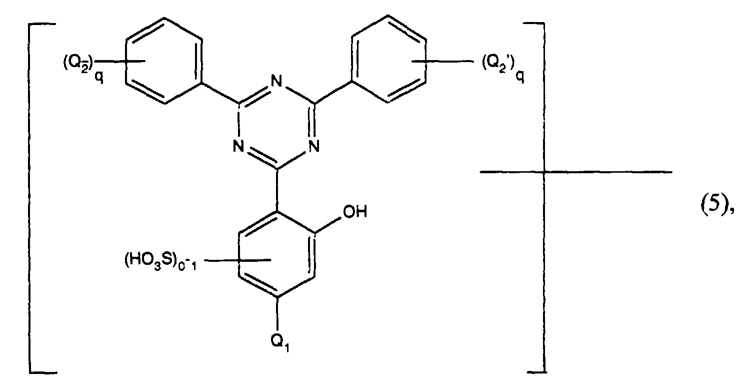 worin q eine ganze Zahl von 1 bis 3 bedeutet und Q₁, Q₂ und Q₂' unabhängig voneinander je Wasserstoff, Hydroxyl, Alkyl mit 1 bis 12 Kohlenstoffatomen, Alkoxy mit 1 bis 18 Kohlenstoffatomen oder gegebenenfalls durch Hydroxy substituiertes C₁-C₄-Alkoxy-C₁-C₄-Alkoxy bedeuten,
eines Oxalsäurediamid-UV-Absorbers der Formel 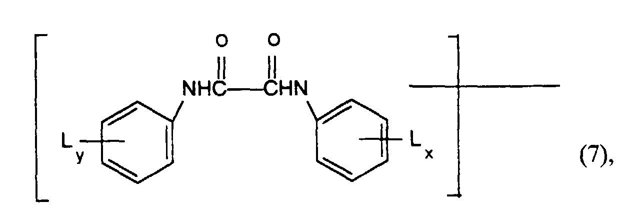 worin x und y unabhängig voneinander je eine ganze Zahl von 0 bis 3 bedeuten, wobei die Summe von (x + y) ≥ 1 ist, und die Substituenten L unabhängig voneinander je Sulfo, unsubstituiertes oder im Alkylteil durch Sulfo substituiertes Alkyl, Alkoxy oder Alkylthio mit je 1 bis 22 Kohlenstoffatomen oder unsubstituiertes oder im Phenylring durch Sulfo substituiertes Phenoxy oder Phenylthio bedeuten,
eines Acrylat-UV-Absorbers oder der Rest einer gegebenenfalls durch Hydroxy oder C₁-C₄-Alkyl substituierten Benzoesäure oder deren Phenyl-, C₁-C₈-Alkylphenyl- oder C₁-C₁₈-Alkylester ist,
W für -NR₂-, -O- oder -S- steht,
W₁ ein Rest -C(O)O-, -O(O)C-, -C(O)NH- oder -HN(O)C- ist,
X₁ und X₂ unabhängig voneinander je Halogen, Hydroxy, -NH₂, unsubstituiertes oder im Alkylteil durch Hydroxy, Carboxy, Sulfo, Sulfato oder C₁-C₄-Alkoxy substituiertes N-Mono- oder N,N-Di-C₁-C₄-Alkylamino, Cyclohexylamino, unsubstituiertes oder im Phenylteil durch C₁-C₄-Alkyl, C₁-C₄-Alkoxy, Carboxy, Sulfo, Halogen oder durch einen Rest der Formel

-SO₂-Y (8a),

-CONH-(CH₂)ₚ-SO₂-Y (8b),

worin Y Vinyl oder einen Rest -CH₂-CH₂-G bedeutet, G eine Abgangsgruppe ist und p eine ganze Zahl 1 bis 6 bedeutet, substituiertes Phenylamino,
N-C₁-C₄-Alkyl-N-phenylamino, 3-Carboxypyridin-1-yl oder 3-Carbamoylpyridin-1-yl bedeuten, bevorzugt stehen X₁ und X₂ unabhängig voneinander je für Chlor oder Fluor
T unabhängig eine der für X₁ angegebenen Bedeutungen hat oder für einen C₁-C₄- Alkoxyrest, einen gegebenenfalls durch C₁-C₄-Alkyl, C₁-C₄-Alkoxy, Halogen, Hydroxy, Carboxy oder Sulfo substituierten Phenoxyrest, einen C₁-C₄-Alkylthiorest oder einen gegebenenfalls durch C₁-C₄-Alkyl, C₁-C₄-Alkoxy, Halogen, Hydroxy, Carboxy oder Sulfo substituierten Phenylthiorest oder für einen Piperidino-, Piperazino- oder Morpholinorest steht oder unabhängig ein Rest U-(B₂)ᵣ-(W₁)ₛ-(B₁)ₜ-W-, worin U, B₁, B₂, W und W₁ jeweils die oben angegebene Bedeutung haben, ist, und
r, s und t unabhängig voneinander je die Zahl 0 oder 1 bedeuten, wobei s 0 bedeutet, wenn t 0 ist,
mit der Massgabe, dass die Verbindungen der Formel (1) mindestens eine Sulfo- oder Sulfatogruppe und mindestens eine alkalisch abspaltbare Gruppe aufweisen.

B steht bevorzugt für geradkettiges oder verzweigtes C₂-C₆-Alkylen, welches gegebenenfalls durch Hydroxy, Sulfo oder Sulfato substituiert ist. Beispiele für besonders bevorzugte Alkylenreste B sind 1,2-Ethylen, 1,2-Propylen, 1,3-Propylen, 2-Hydroxy-1,3-Propylen, 1,4-Butylen, 2-Methyl-1,5-pentylen und 1,6-Hexylen.

Falls -NR- und -NR'- zusammen einen heterocyclischen Ring bilden, handelt es sich z.B. um einen Piperazinring.

Bevorzugt als aromatisches Brückenglied B sind unsubstituiertes oder durch Sulfo, Carboxy, Chlor, Methyl oder Methoxy substituiertes 1,3- oder 1,4-Phenylen, durch 1 oder 2 Sulfogruppen substituiertes Naphthylen oder ein Rest der Formel 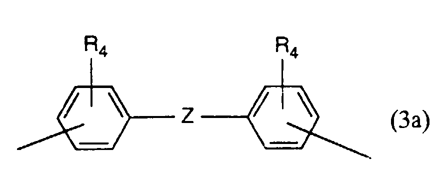 oder 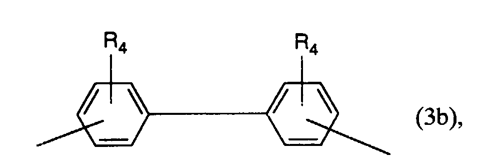 worin Z -NHCONH-, -O-, -NH-, -CH=CH- oder -CH₂- und R₄ Wasserstoff oder Sulfo bedeuten.

Beispiele für besonders bevorzugte aromatische Brückenglieder B sind 1,3-Phenylen, 1,4-Phenylen, 4-Methylphenylen-1,3, 4-Sulfophenylen-1,3, 3-Sulfophenylen-1,4, 3,6-Disulfophenylen-1,4, 4,6-Disulfophenylen-1,3, 3,7-Disulfonaphthylen-1,5, 4,8-Disulfonaphthylen-2,6, 2,2'-Disulfodiphenylen-4,4', 4,4'Phenylenharnstoff-2,2'-disulfonsäure oder 2,2'-Disulfostilbenylen-4,4' und insbesondere 4-Sulfophenylen-1,3, 3-Sulfophenylen-1,4, 3,6-Disulfophenylen-1,4 oder 4,6-Disulfophenylen-1,3.

Beispiele für aromatisch-aliphatische Brückenglieder B sind unsubstituiertes oder im Phenylenteil z.B. durch Sulfo, Methyl, Methoxy, Carboxy oder Chlor substituiertes Phenylen-C₁-C₄-Alkylen. Bevorzugt steht B als aromatisch-aliphatisches Brückenglied für unsubstituiertes oder im Phenylenteil durch Sulfo, Methyl oder Methoxy substituiertes Phenylenmethylen.

B bedeutet bevorzugt C₂-C₆-Alkylen, welches gegebenenfalls durch Hydroxy, Sulfo oder Sulfato substituiert ist, unsubstituiertes oder durch Sulfo, Carboxy, Chlor, Methyl oder Methoxy substituiertes 1,3- oder 1,4-Phenylen, durch 1 oder 2 Sulfogruppen substituiertes Naphthylen oder einen Rest der Formel 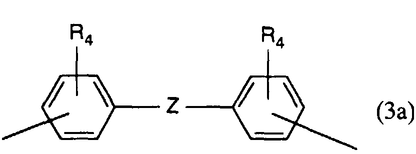 oder 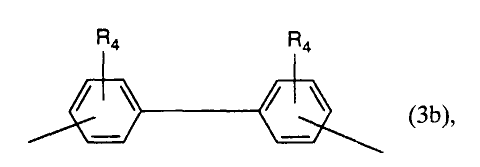 worin Z -NHCONH-, -O-, -NH-, -CH=CH- oder -CH₂- und R₄ Wasserstoff oder Sulfo sind.

Besonders bevorzugt steht B für 4-Sulfophenylen-1,3, 3-Sulfophenylen-1,4, 3,6-Disulfophenylen-1,4 oder 4,6-Disulfophenylen-1,3.

Bei B₁ oder B₂ als geradkettiges oder verzweigtes C₁-C₁₂-Alkylen handelt es sich bevorzugt um geradkettiges oder verzweigtes C₁-C₆-Alkylen. Beispiele für besonders bevorzugte Alkylenreste B₁ und B₂ sind Methylen, 1,2-Ethylen, 1,2-Propylen, 1,3-Propylen, 1,4-Butylen 2-Methyl-1,5-pentylen und 1,6-Hexylen und insbesondere Methylen und 1,2-Ethylen.

r und s stehen bevorzugt jeweils für die Zahl 0.
R, R' und R₂ bedeuten vorzugsweise unabhängig voneinander je Wasserstoff oder C₁-C₄-Alkyl und insbesondere bevorzugt Wasserstoff, Methyl oder Ethyl.

Beispiele für geeigenete UV-Absorber-Reste U der Formel (4) sind der Rest von 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5'-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl- 2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tert-amyl-2'-hydroxyphenyl)-benzotriazol, 2-(3',5'-Bis-(α,α-dimethylbenzyl)-2'-hydroxyphenyl)-benzotriazol, Mischung aus 2-(3'-tert-Butyl-2'-hydroxy-5'-(2-octyloxycarbonylethyl)phenyl)-5-chlorbenzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol, und 2-(3'-tert-Butyl-2'-hydroxy-5'-(2-isooctyloxycarbonylethyl)phenyl-benzotriazol, 2,2'-Methylen-bis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300, 2-(2'-Hydroxy-5'-sulfophenyl)-benzotriazol, 2-(2'-Hydroxy-5'-methylphenyl)-5-sulfobenzotriazol, oder von 2-(2'-Hydroxy-5'-tert.-butylphenyl)-5-sulfobenzotriazol.

Besonders bevorzugt steht U als 2-Hydroxyphenylbenztriazolrest für einen Rest der Formel 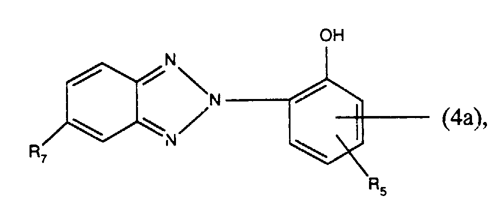 worin R₅ Wasserstoff, C₁-C₄-Alkyl oder Sulfo und R₇ Wasserstoff, Sulfo oder Carboxy bedeuten, und worin einer der Reste R₅ und R₇ verschieden von Wasserstoff ist.

Beispiele für geeignete 2-Hydroxyphenyltriazin-Reste U sind der Rest von 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-(2-Hydroxy-4-methoxy-6-sulfophenyl)4,6-bis(phenyl)-1,3,5-triazin oder 2-[2-hydroxy-4-(2-hydroxy-3-octyloxypropyloxy)phenyl]-4,6-bis(2,4-dimethylphenyl)-1,3,5-triazin.

Beispiele für geeignete 2-Hydroxybenzophenon-Reste U sind der Rest von 2,4-Dihydroxy-, 2-Hydroxy-4-methoxy-, 2-Hydroxy-4-octoxy-, 2-Hydroxy-4-decyloxy-, 2-Hydroxy-4-dodecyloxy-, 2-Hydroxy-4-methoxy-5-sulfo-, 2-Hydroxy-4-benzyloxy-, 4,2',4'-Trihydroxy- oder 2'-Hydroxy-4,4'-dimethoxybenzophenon.

Steht U für den Rest eines 2-Hydroxybenzophenons, so entspricht dieser bevorzugt der Formel 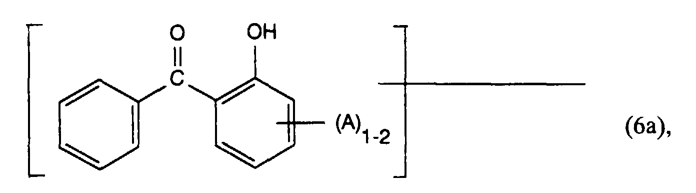 worin (A)₁₋₂ für 1 oder 2 gleiche oder verschiedene Reste aus der Gruppe C₁-C₁₂-Alkoxy und Sulfo steht.

Beispiele für geeignete Oxalanilidreste U sind der Rest von 4,4'-Di-octyloxy-oxanilid, 2,2'-Diethoxy-oxanilid, 2,2'-Di-octyloxy-5,5'-di-tert-butyl-oxanilid, 2,2'-Di-dodecyloxy-5,5'di-tert-butyl-oxanilid, 2-Ethoxy-2'-ethyl-oxanilid, 2-Methoxy-5-sulfooxanilid, 2-Ethoxy-5-sulfooxanilid, 2,5-Dimethoxyoxanilid, 2-Ethoxy-5-tert-butyl-2'-ethyloxanilid gegebenenfalls im Gemisch mit dem Rest von 2-Ethoxy-2'-ethyl-5,4'-di-tert-butyloxanilid, oder Gemische der Reste von o- und p-Methoxy- sowie von o- und p-Ethoxy-di-substituierten Oxaniliden.

U steht als Oxanilidrest bevorzugt für einen Rest der Formel 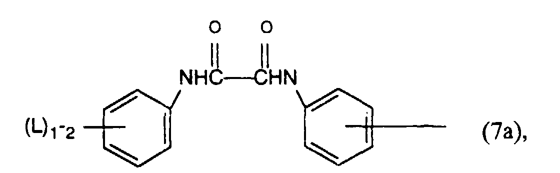 worin (L)₁₋₂ für 1 oder 2 Reste L aus der Gruppe Sulfo, C₁-C₄-Alkyl und C₁-C₁₂-Alkoxy steht.

Geeignete Acrylatreste U sind Acrylsäure-C₁-C₁₀-Alkylester, die in der α-Position unsubstituiert oder durch Cyano oder Carbo-C₁-C₄-Alkoxy substituiert sind, in der einen β-Position einen Phenyl-, C₁-C₄-Alkoxyphenyl oder Indolinylrest tragen und in der anderen β-Position unsubstituiert oder durch Phenyl, C₁-C₄-Alkoxyphenyl oder C₁-C₄-Alkyl substituiert sind.

Beispiele für Acrylatreste U sind der Rest von α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester oder N-(β-Carbomethoxy-β-cyanovinyl)-2-methyl-indolin.

Beispiele für U als Rest einer gegebenenfalls durch Hydroxy oder C₁-C₄-Alkyl substituierten Benzoesäure oder deren Phenyl-, C₁-C₈-Alkylphenyl- oder C₁-C₁₈-Alkylester, sind der Rest von Benzoesäure, 4-tert-Butyl-phenylsalicylat, Phenylsalicylat, Octylphenyl-salicylat, Dibenzoylresorcin, Bis-(4-tert-butylbenzoyl)resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester oder 3,5-Di-tert-butyl-4-hydroxybenzoesäure-2-methyl-4,6-di-tert-butylphenylester.

U bedeutet bevorzugt einen Rest der zuvor angegebenen Formel (4a), (6a) oder (7a) oder den Rest eines unsubstituierten oder durch Hydroxy oder C₁-C₄-Alkyl substituierten Benzoesäurerests oder dessen Phenyl-, C₁-C₈-Alkylphenyl- oder C₁-C₁₈-Alkylesters.

W steht bevorzugt für einen Rest -NR₂-, worin für R₂ die zuvor genannten Bedeutungen und Bevorzugungen gelten. Insbesondere Bevorzugt bedeutet W den Rest -NH-.

Geeignete Abgangsgruppen G sind z.B. Halogen, z.B. Chlor, Acyloxy, z.B. Acetoxy oder Benzoyloxy, Phosphato, Sulfato und Thiosulfato.

Beispiele für geeignete Reste Y sind dementsprechend Vinyl, β-Brom- und β-Chlorethyl, β-Acetoxyethyl, β-Benzoyloxyethyl, β-Phosphatoethyl, β-Sulfatoethyl und β-Thiosulfatoethyl. Y steht bevorzugt für Vinyl oder β-Sulfatoethyl.

p bedeutet bevorzugt die Zahl 2, 3 oder 4 und besonders bevorzugt die Zahl 2 oder 3.

X₁ und X₂ stehen unabhängig voneinander je bevorzugt für Amino, unsubstituiertes oder im Alkylteil durch Hydroxy, Sulfo oder Sulfato substituiertes N-Mono- oder N,N-Di-C₁-C₄-Alkylamino, wie z.B. Methylamino, Ethylamino, Carboxymethylamino, β-Hydroxyethylamino, β-Sulfoethylamino, N,N-Di-β-hydroxyethylamino, Carboxymethylamino, Cyclohexylamino, unsubstituiertes oder im Phenylteil durch Methyl, Ethyl, Methoxy, Ethoxy, Carboxy, Sulfo, Chlor oder durch einen Rest der Formel (8a) oder (8b) substituiertes Phenylamino, wie z.B. o-, m- oder p-Methylphenylamino, o-, m- oder p-Methoxyphenylamino, o-, m- oder p-Sulfophenylamino, 2,4- oder 2,5-Disulfophenylamino, o-Carboxyphenylamino, N-Ethyl-N-phenylamino, N-Methyl-N-phenylamino oder für Phenylamino, welches durch einen Rest der Formel (8a) oder (8b) substituiert ist.

Besonders bevorzugt sind für X₁ und X₂ unabhängig voneinander je die Bedeutungen Chlor, Fluor, Amino, β-Sulfoethylamino, β-Hydroxyethylamino, N,N-Di-β-hydroxyethylamino und o-, m- oder p-Sulfophenylamino.

Bedeutet T einen C₁-C₄-Alkoxyrest, handelt es sich bevorzugt um Methoxy, Ethoxy, n- oder iso-Propoxy oder n-, iso-, sec.- oder tert.-Butoxy; bevorzugt sind hierbei die Bedeutungen Methoxy und iso-Propoxy.

T steht als C₁-C₄-Alkylthio bevorzugt für Methyl- oder Ethylthio.

Hat T unabhängig eine der zuvor für X₁ angegebenen Bedeutungen, so gelten hierbei die zuvor genannten Bevorzugungen.

T steht bevorzugt für -NH₂, unsubstituiertes oder im Alkylteil durch Hydroxy, Carboxy, Sulfo, Sulfato oder C₁-C₄-Alkoxy substituiertes N-Mono- oder N,N-Di-C₁-C₄-Alkylamino, Cyclohexylamino, unsubstituiertes oder im Phenylteil durch C₁-C₄-Alkyl, C₁-C₄-Alkoxy, Carboxy, Sulfo, Halogen oder durch einen Rest der Formel

-SO₂-Y (8a),

-CONH-(CH₂)ₚ-SO₂-Y (8b),

worin Y Vinyl oder einen Rest -CH₂-CH₂-G bedeutet, G eine Abgangsgruppe ist und p eine ganze Zahl 1 bis 6 bedeutet, substituiertes Phenylamino, N-C₁-C₄-Alkyl-N-phenylamino, Morpholino, C₁-C₄-Alkoxy oder für einen Rest -W-(B₁)ₜ-(W₁)ₛ-(B₂)ᵣ-U, worin für W, W₁, B₁, B₂, U, r, s und t die zuvor angegebenen Bedeutungen und Bevorzugungen gelten.

T steht besonders bevorzugt für einen Rest -W-(B₁)ₜ-U, worin für W, B₁, U und t die zuvor angegebenen Bedeutungen und Bevorzugungen gelten, oder für Amino, Methylamino, Ethylamino, Carboxymethylamino, β-Hydroxyethylamino, β-Sulfoethylamino, N,N-Di-β-hydroxyethylamino, Cyclohexylamino, o-, m- oder p-Methylphenylamino, o-, m- oder p-Methoxyphenylamino, o-, m- oder p-Sulfophenylamino, 2,4- oder 2,5-Disulfophenylamino, o-Carboxyphenylamino, N-Ethyl-N-phenylamino, N-Methyl-N-phenylamino oder für Phenylamino, welches durch einen Rest der zuvor angegebenen Formel (8a) oder (8b) substituiert ist.

Insbesondere bevorzugt steht T für einen Rest -W-(B₁)ₜ-U, worin für W, B₁, U und t die zuvor angegebenen Bedeutungen und Bevorzugungen gelten.

Eine bevorzugte Ausführungsform der vorliegenden Erfindung betrifft Verbindungen der Formel 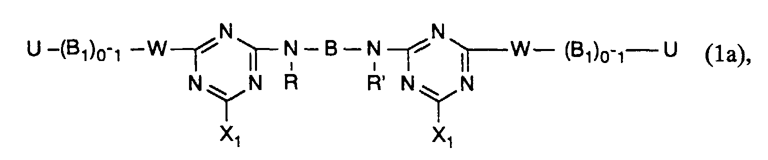 worin B 4-Sulfophenylen-1,3, 3-Sulfophenylen-1,4, 3,6-Disulfophenylen-1,4 oder 4,6-Disulfophenylen-1,3 bedeutet, B₁ geradkettiges oder verzweigtes C₁-C₆-Alkylen ist, R und R' unabhängig voneinander je Wasserstoff, Methyl oder Ethyl bedeuten, W für die Gruppe -NH- steht, X₁ und X₂ jeweils Chlor oder Fluor bedeuten, und U
(i) einen Rest der Formel 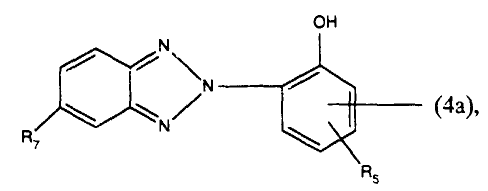 worin R₅ Wasserstoff, C₁-C₄-Alkyl oder Sulfo und R₇ Wasserstoff, Sulfo oder Carboxy bedeuten, und worin einer der Reste R₅ und R₇ verschieden von Wasserstoff ist;
(ii) einen Rest der Formel 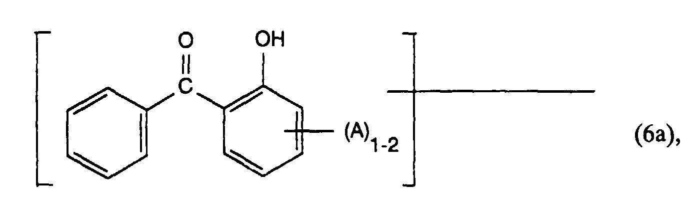 worin (A)₁₋₂ für 1 oder 2 gleiche oder verschiedene Reste aus der Gruppe C₁-C₁₂-Alkoxy und Sulfo steht;
(iii) einen Rest der Formel 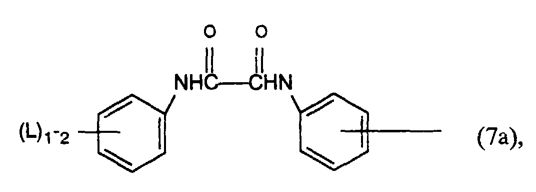 worin (L)₁₋₂ für 1 oder 2 Reste L aus der Gruppe Sulfo, C₁-C₄-Alkyl und C₁-C₁₂-Alkoxy steht; oder
(iv) einen unsubstituierten oder durch Hydroxy oder C₁-C₄-Alkyl substituierten Benzoesäurerest oder dessen Phenyl-, C₁-C₈-Alkylphenyl- oder C₁-C₁₈-Alkylester bedeutet.

Eine besonders bevorzugte Ausführungsform der vorliegenden Erfindung betrifft Verbindungen der zuvor angegebenen Formel (1a), worin B, B₁, R, R', W und X₁ jeweils die unter der Formel (1a) angegebene Bedeutung haben und U ein Rest der zuvor angegebenen Formel (7a) ist.

In den Formeln (1) bis (8b) bedeuten C₁-C₁₈-Alkyl generell z.B. Methyl, Ethyl, n- oder iso-Propyl, n-, iso-, sec- oder tert-Butyl oder geradkettiges oder verzweigtes Pentyl, Hexyl, Heptyl, Octyl, Nonyl, Decyl, Undecyl, Dodecyl, Tridecyl, Tetradecyl, Pentadecyl, Hexadecyl, Heptadecyl oder Oktadecyl. Unter C₁-C₁₈-Alkoxy ist generell z.B. Methoxy, Ethoxy, n- oder iso-Propoxy, n-, iso-, sec- oder tert-Butoxy oder geradkettiges oder verzweigtes Pentoxy, Hexyloxy, Heptyloxy, Octyloxy, Nonyloxy, Decyloxy, Undecyloxy, Dodecyloxy, Tridecyloxy, Tetradecyloxy, Pentadecyloxyl, Hexadecyloxy, Heptadecyloxy oder Oktadecyloxy zu verstehen. Beispiele für C₁-C₁₂-Alkylen sind Methylen, 1,1- oder 1,2-Ethylen, 1,2- oder 1,3-Propylen oder geradkettiges oder verzweigtes Butylen, Pentylen, Hexylen, Heptylen, Oktylen, Nonylen, Decylen, Undecylen oder Dodecylen. Halogen steht generell z.B. für Fluor, Chlor oder Brom. C₁-C₄-Alkoxycarbonyl bedeutet generell z.B. Methoxycarbonyl, Ethoxycarbonyl, n- oder iso-Propoxycarbonyl oder n-, iso-, sec- oder tert-Butoxycarbonyl. Beispiele für C₁-C₄-Alkylthio sind Methyl- oder Ethylthio. Beispiele für C₅-C₈-Cycloalkyl sind Cyclopentyl oder insbesondere Cyclohexyl.

Die Verbindungen der Formel (1) müssen mindestens eine alkalisch abspaltbare Gruppe, d.h. z.B. mindestens ein Halogenatom an einem Triazinylrest oder einen Rest der zuvor angegebenen Formel (8a) oder (8b) aufweisen.

Die Verbindungen der Formel (1) müssen ferner mindestens eine Sulfo- oder Sulfatogruppe aufweisen, wobei diese jeweils in Form der freien Säure oder vorzugsweise in Salzform, z.B. als Na-, Li-, K- oder Ammoniumsalz vorliegen kann.

Die Verbindungen der Formel (1) sind faserreaktiv. Unter faserreaktiven Resten sind solche zu verstehen, die mit den Hydroxygruppen der Cellulose, den Amino-, Carboxy-, Hydroxy- und Thiolgruppen bei Wolle und Seide, oder mit den Amino- und eventuell Carboxygruppen von synthetischen Polyamiden unter Bildung kovalenter chemischer Bindungen zu reagieren vermögen.

Die Verbindungen der Formel (1) können hergestellt werden, z.B. indem man eine Verbindung der Formel

U-(B₂)ᵣ-(W₁)ₛ-(B₁)ₜ-W-H (8)

eine Verbindung der Formel

H-RN-B-NR'-H (9),

eine Verbindung der Formel 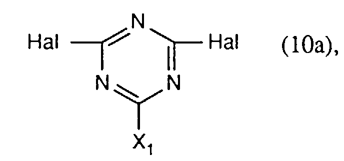 eine Verbindung der Formel 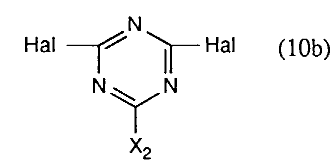 und
gegebenenfalls eine Verbindung der Formel

T*-H (11),

worin U, B, B₁, B₂, W, W₁, R, R', X₁, X₂, r, s und t jeweils die zuvor angegebene Bedeutung haben, Hal Halogen, vorzugsweise Fluor oder Chlor, bedeutet und T* die zuvor für T angegebene Bedeutung ausgenommen Halogen hat, miteinander umsetzt, wobei die Reihenfolge der Teilreaktionen unter Berücksichtigung der miteinander umzusetzenden Ausgangsverbindungen frei gewählt werden kann.

Eine Variante des Verfahrens für den Fall, dass r und s jeweils die Zahl 0 darstellen, T einen Rest -W-(B₁)ₜ-U bedeutet und X₁ und X₂ identisch sind, besteht darin, dass man in etwa 1 Moläquivalent einer Verbindung der Formel

U-(B₁)ₜ-W-H (8')

zunächst mit in etwa 1 Moläquivalent einer Verbindung der Formel 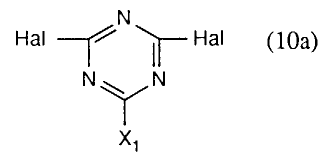 umsetzt, und das gebildete primäre Kondensationsprodukt anschliessend mit in etwa 0,5 Moläquivalenten eines Diamins der Formel

H-RN-B-NR'-H (9)

umsetzt, worin Hal, X₁, R, R', B, U, B₁, W und t jeweils die zuvor angegebene Bedeutung haben.

Eine weitere Variante des Verfahrens für den Fall, dass r und s jeweils die Zahl 0 und T einen Rest -W-(B₁)ₜ-U bedeuten, besteht darin, dass man in etwa 2 Moläquivalente einer Verbindung der Formel

U-(B₁)ₜ-W-H (8')

mit in etwa 1 Moläquivalent einer Verbindung der Formel 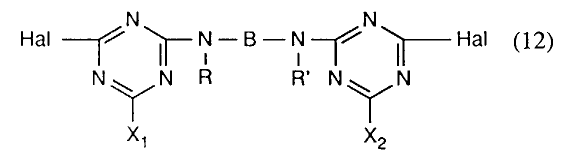 umsetzt, worin Hal, X₁, X₂, R, R', B, U, B₁, W und t jeweils die zuvor angegebene Bedeutung haben.

Die bei der Kondensation der Verbindungen der Formel (8), (9) und (11) mit einer Halotriazinverbindung anzuwendenden Bedingungen sind aus dem Bereich der Reaktivfarbstoffchemie hinlänglich bekannt. Üblicherweise verlaufen diese Reaktion in einem wässrigen oder wässrig-organischen Medium in Gegenwart von säurebindenden Mitteln, z.B. Natriumcarbonat oder Natriumhydroxid.

Die Verbindungen der Formel (9), (10a), (10b), (11) und (12) sind bekannt oder können nach an sich bekannten Methoden erhalten werden.

Die UV-Absorber-Verbindungen der Formel (8) bzw. (8') gehören bekannten Substanzklassen an und lassen sich in an sich bekannter Weise, z.B. wie in der US-A 3,041,330, US-A 3,042,669 oder US-A 3,159,646 beschrieben, herstellen.

Die neuen UV-Absorber der Formel (1) eignen sich zum photochemischen Stabilisieren von ungefärbten und gefärbten oder bedruckten Fasermaterialien z.B. aus Seide, Leder, Wolle, Polyamid oder Polyurethanen, und insbesondere von cellulosehaltigen Fasermaterialien aller Art. Solche Fasermaterialien sind beispielsweise die natürliche Cellulosefaser, wie Baumwolle, Leinen, Jute und Hanf, sowie Zellstoff und regerierte Cellulose. Bevorzugt sind textile Fasermaterialien aus Baumwolle. Die Verbindungen der Formel (1) eignen sich auch zum photochemischen Stabilisieren von hydroxylgruppenhaltigen Fasern, die in Mischgeweben enthalten sind, z.B. von Gemischen aus Baumwolle mit Polyesterfasern oder Polyamidfasern. Ein weiteres bevorzugtes Anwendungsgebiet betrifft die Sperrung bzw. Verminderung der durch die genannten Textilmaterialien fallenden UV-Strahlung (UV-Cutting) und den erhöhten Sonnenschutz, den mit einer erfindungsgemässen Verbindung ausgerüstete Textilmaterialien der menschlichen Haut bieten.

Dazu werden eine oder mehrere verschiedene Verbindungen der Formel (1) vorteilhaft in einer Menge von 0,01 bis 5 Gew.-%, vorzugsweise 0,1-3, und insbesondere 0,25 bis 2 Gew.-%, bezogen auf das Gewicht des Fasermaterials, nach einem der üblichen Färbeverfahren für Reaktivfarbstoffe auf das textile Fasermaterial aufgebracht. Handelt es sich bei dem Textilfasermaterial um ein mit einem Reaktivfarbstoff gefärbtes cellulosisches Material, so kann das Applizieren des UV-Absorbers der Formel (1) vor, während oder nach der Färbung, vorzugsweise gleichzeitig mit der Applikation des Farbstoffs erfolgen.

Die erfindungsgemässen Verbindungen der Formel (1) lassen sich auf verschiedene Weise auf das Fasermaterial applizieren und auf der Faser fixieren, insbesondere in Form von wässrigen oder wässrig-organischen Lösungen oder Druckpasten. Sie eignen sich sowohl für das Ausziehverfahren als auch für die Foulardfärberei, können bei niedrigen Temperaturen eingesetzt werden und erfordern hei Pad-Steam-Verfahren nur kurze Dämpfzeiten. Die Fixiergrade sind hoch, und die nicht-fixierten Anteile können leicht ausgewaschen werden wobei die Differenz zwischen Ausziehgrad und Fixiergrad bemerkenswert klein ist. Die Verbindungen der Formel (1) eignen sich auch zum Druck, vor allem auf Baumwolle.

Die mit den erfindungsgemässen Verbindungen der Formel (1) ausgerüsteten textilen Fasermaterialien besitzen einen verbesserten Schutz gegen photochemischen Faserabbau und Vergilbungserscheinungen sowie im Fall von gefärbtem Fasermaterial eine verbesserte (Heiss-)Lichtechtheit. Besonders hervorzuheben ist die stark verbesserte Lichtschutzwirkung des behandelten textilen Fasermaterials, die dadurch zum Ausdruck kommt, dass das mit einer erfindungsgemässen Verbindung der Formel (1) ausgerüstete textile Fasermaterial einen gegenüber dem unbehandelten Gewebe stark erhöhten Sonnenschutzfaktor (SF) aufweist.
Der Sonnenschutzfaktor wird definiert als Quotient aus schädlicher UV-Strahlungsdosis ohne Sonnenschutz und schädlicher UV-Strahlungsdosis mit Sonnenschutz.
Dementsprechend stellt ein Sonnenschutzfaktor auch einen Massstab für die Durchlässigkeit der unbehandelten und der mit einer erfindungsgemässen Verbindung der Formel (1) behandelten Fasermaterialien für UV-Strahlung dar. Die Bestimmung des Sonnenschutzfaktors von textilen Fasermaterialien wird z.B. in der WO 94/04515 oder in J. Soc. Cosmet. Chem. 40, 127-133 (1989) erläutert und kann analog dazu erfolgen.

Die nachfolgenden Beispiele dienen der Erläuterung der Erfindung. Die Temperaturen sind in Celsiusgraden angegeben, Teile sind Gewichtsteile, die Prozentangaben beziehen sich auf Gewichtsprozente, sofern nichts anderes vermerkt ist.

Beispiel 1: Ein Gemisch aus 334 Teilen 2-(2'-Hydroxy-5'-tert.-butylphenyl)-benztriazol und 193 Teilen N-Hydroxymethylchloracetamid wird bei Raumtemperatur in 1380 Teile 98%ige Schwefelsäure eingetragen. Man lässt ca. drei Stunden bei Raumtemperatur nachrühren und trägt dann das Reaktionsgemisch auf ein Eis/Wasser-Gemisch aus. Der erhaltene Niederschlag der Verbindung der Formel 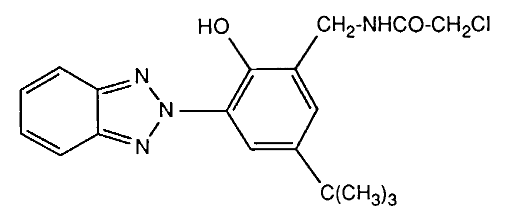 wird abfiltriert, mit Wasser gewaschen und getrocknet.

Beispiel 2: Eine Lösung von 223 Teilen der gemäss Beispiel 1 erhaltenen Benztriazolverbindung werden ca. 20 Stunden lang in 745 Teilen 100%iger Schwefelsäure bei einer Temperatur von 80-82°C und einem Druck von ca. 130 mbar gehalten. Nach dem Abkühlen wird das Reaktionsgemisch auf Eis/Wasser ausgetragen. Man gibt anschliessend 45 Teile Kieselgel hinzu, rührt das Ganze ca. 30 Minuten bei 40°C und führt eine Klärfiltration durch. Das gesammelte Filtrat wird ca. 3,5 Stunden auf 95°C erhitzt und der nach dem Abkühlen anfallende, die Verbindung der Formel 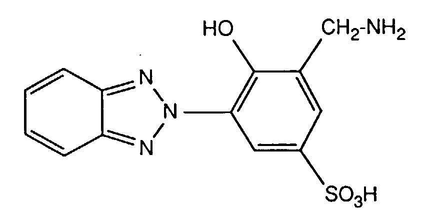 enthaltende Niederschlag abfiltriert und mit Wasser gewaschen.

Zur Reinigung wird der erhaltene Feststoff in 1000 Teilen Wasser bei einer Temperatur von 35°C verrührt und der pH mit konz. Natriumhydroxidlösung auf einen Wert von 11 gestellt. Nach Zugabe von Kieselgel und Erhitzen auf ca. 80°C wird der unlösliche Rückstand heiss abfiltriert und mit heissem Wasser ausgewaschen. Man stellt das gesammelte Filtrat mit 50%iger Schwefelsäure kongosauer und fällt so das Produkt aus, welches anschliessend abfiltriert, mit Wasser neutral gewaschen und getrocknet wird.

Das Rohprodukt wird in Wasser/N,N-Dimethylformamid/Dioxan aufgenommen und durch Zugabe von 30%igem Ammoniak gelöst. Danach versetzt man langsam mit 4 n Salzsäure und fällt so das Produkt erneut aus. Dieses wird anschliessend abfiltriert, mit Wasser gewaschen und im Vakuum getrocknet.

Beispiel 3: Man bringt 6,4 Teile der gemäss Beispiel 2 erhaltenen sulfonierten Benztriazolverbindung in 200 Teilen Wasser und 140 Teilen Dimethoxyethan unter Zugabe von 10 Teilen 2 n Natriumhydroxidlösung in Lösung und trägt innerhalb von ca. 2 Stunden bei einer Temperatur von ca. 35°C 5,6 Teile der Verbindung der Formel 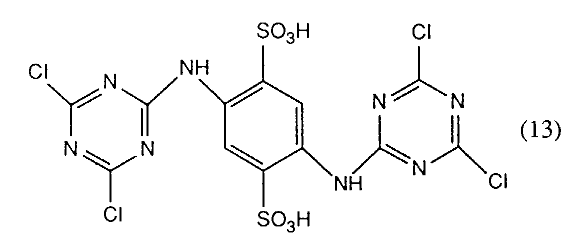 (Herstellung gemäss DE-OS 2,105,309, Beispiel 2) ein; dabei wird der pH-Wert durch Zugabe von 2 n Natriumhydroxidlösung konstant bei 8,5 gehalten. Nach einem 30 minütigen Nachrühren stellt man den pH auf 7,5 ein und lässt ca. weitere 15 Stunden bei 35°C nachrühren.

Anschliessend wird das Reaktionsgemisch klärfiltriert und die Verbindung der Formel 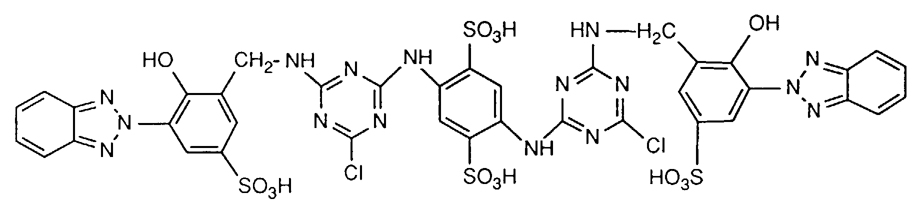 durch Aussalzen isoliert. Bringt man die Verbindung mittels eines üblichen Färbeverfahrens für Reaktivfarbstoffe auf ein Baumwollgewebe auf, so weist dieses einen gegenüber der unbehandelten Ware stark erhöhten Sonnenschutzfaktor auf.

Beispiel 4: Zu einer Lösung von 3 Teilen der Verbindung im im Beispiel 3 angegebenen Formel (13) in 60 Teilen Wasser werden 1,5 Teile 4-Aminobenzoesäure gegeben und das Gemisch unter Konstanthaltung des pH-Werts bei 7 mittels Zudosierung von Natriumhydroxidlösung gerührt. Nach beendeter Reaktion wird die Verbindung der Formel 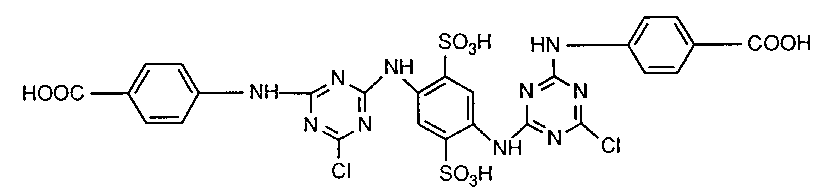 durch Aussalzen isoliert. Bringt man die Verbindung mittels eines üblichen Färbeverfahrens für Reaktivfarbstoffe auf ein Baumwollgewebe auf, so weist dieses einen gegenüber der unbehandelten Ware stark erhöhten Sonnenschutzfaktor auf.

Beispiel 5: Ein Gemisch aus 20 Teilen 3-Nitrooxanilsäureethylester und 23 Teilen o-Phenetidin wird 4 Stunden lang unter Stickstoffatmosphäre auf 140-150°C erhitzt. Danach wird der Druck auf ca. 40 mbar abgesenkt und das Gemisch weitere 4 Stunden bei ca. 140°C gerührt. Man kühlt ab und versetzt den halbfesten Rückstand mit Wasser und 4 n Salzsäure. Dann wird die Verbindung der Formel 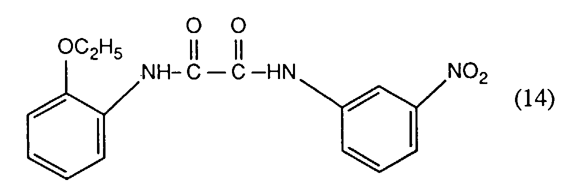 abfiltriert, mit Wasser gewaschen und getrocknet.

Beispiel 6: Zu 55 Teilen 100%iger Schwefelsäure trägt man innerhalb einer Stunde 9,9 Teile der Verbindung der Formel (14) gemäss Beispiel 5 ein und hält dabei die Temperatur unterhalb von 20°C. Anschliessend lässt man eine Stunde weiterrühren und trägt dann auf Eis aus. Nach Zugabe von Kochsalz wird der erhaltene Feststoff abfiltriert, anschliessend in Kochsalzlösung wieder aufgenommen und durch Behandeln mit Natriumhydroxidlösung das Produkt der Formel 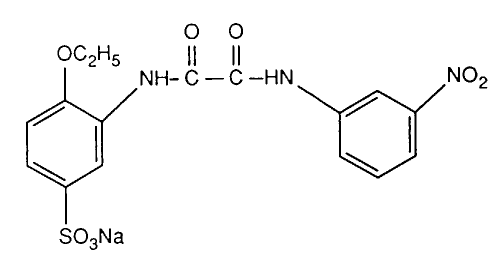 gewonnen.

Beispiel 7: Eine Anschlämmung von 12 Teilen der gemäss Beispiel 6 erhaltenen Nitroverbindung in 1000 ml Wasser wird mittels eines Pd/C (5%)-Katalysators katalytisch hydriert und die erhaltene Verbindung der Formel 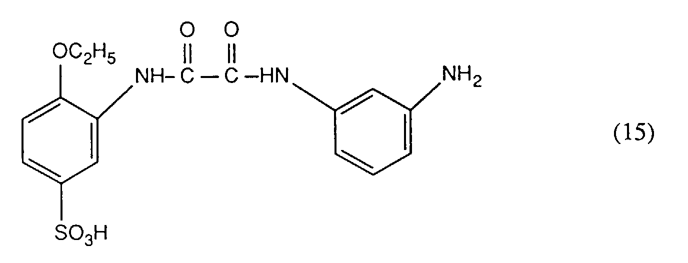 anschliessend in üblicher Weise isoliert und gereinigt.

Beispiel 8: Zu einer Anschlämmung von 2,9 Teilen der gemäss Beispiel 3 erhaltenen Verbindung der Formel (13) in 75 Teilen Wasser werden 4 Teile der gemäss Beispiel 7 erhaltenen Oxalanilidkomponente eingetragen. Man erwärmt das Reaktionsgemisch allmählich unter Rühren auf 35°C und hält den pH-Wert durch Zutropfen von Natriumhydroxidlösung bei ca. 7 konstant. Nach beendeter Kondensation wird das Reaktionsgemisch mittels Dialyse entsalzt und gefriergetrocknet. Man erhält die Verbindung der Formel 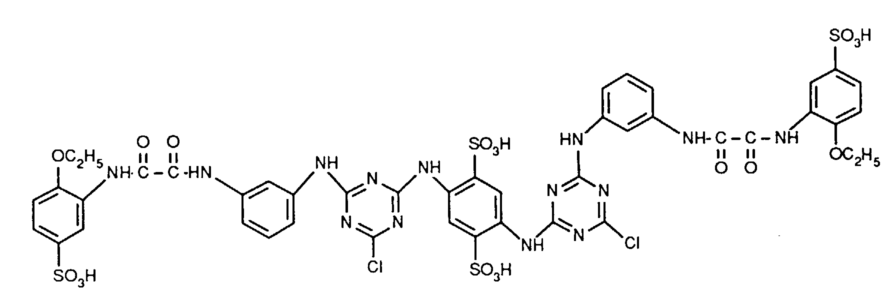 als Pulver. Bringt man die Verbindung mittels eines üblichen Färbeverfahrens für Reaktivfarbstoffe auf ein Baumwollgewebe auf, so weist dieses einen gegenüber der unbehandelten Ware stark erhöhten Sonnenschutzfaktor auf.

Beispiel 9: Zu einer Anschlämmung von 9,1 Teilen der Verbindung der Formel (15) gemäss Beispiel 7 in 45 Teilen Sulfolan werden 2 Teile 100%ige Schwefelsäure eingetragen und der Druck im Reaktionsgefäss auf 200 mbar abgesenkt. Man erhitzt danach während 4 Stunden auf 190°C. Nach dem Abkühlen auf Raumtemperatur wird mit Ethanol versetzt, der Feststoff aus der Suspension abfiltriert und gut mit Ethanol gewaschen. Nach dem Trocknen erhält man die Verbindung der Formel 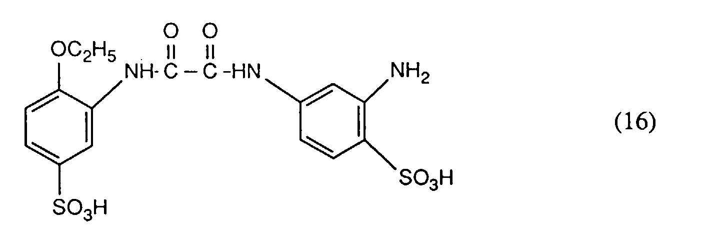 als Pulver.

Beispiele 10-12: Analog wie in den Beispielen 5-7 und 9 beschrieben lassen sich die folgenden Oxanilidverbindungen herstellen.

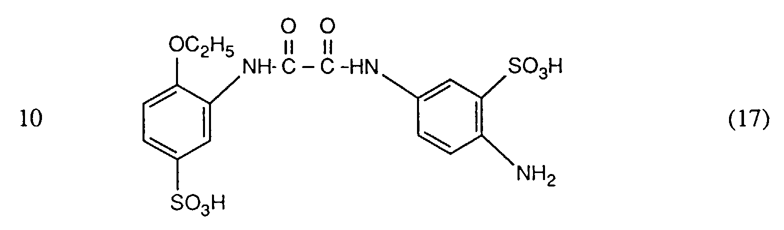

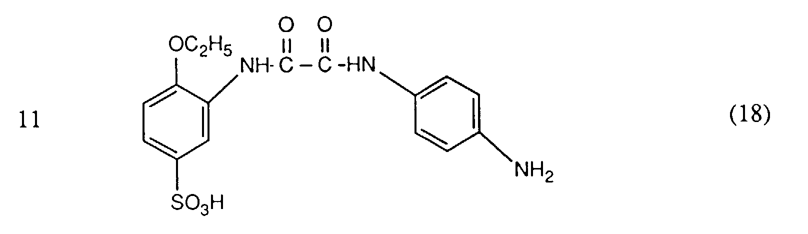

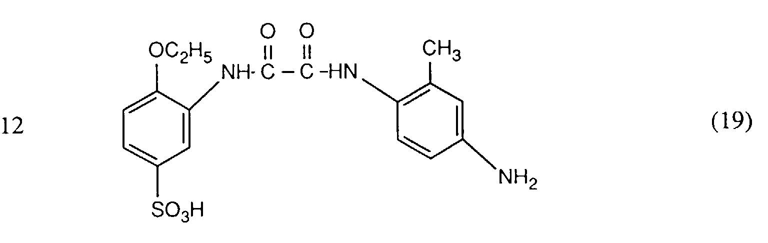

Beispiele 13-16: Analog wie im Beispiel 8 beschrieben lassen sich unter Verwendung der gemäss den Beispielen 9-12 hergestellten Verbindungen der Formeln (16)-(19) die folgenden faserreaktiven UV-Absorber herstellen.

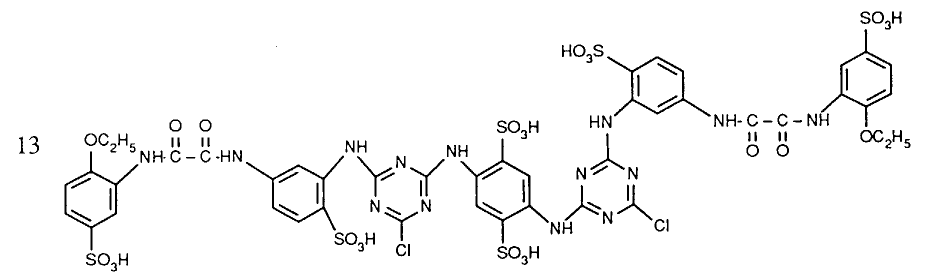

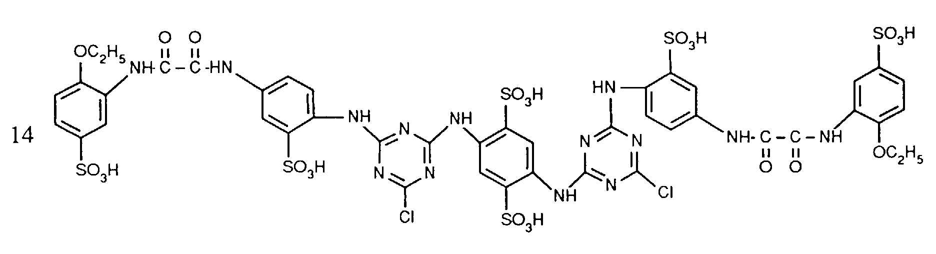

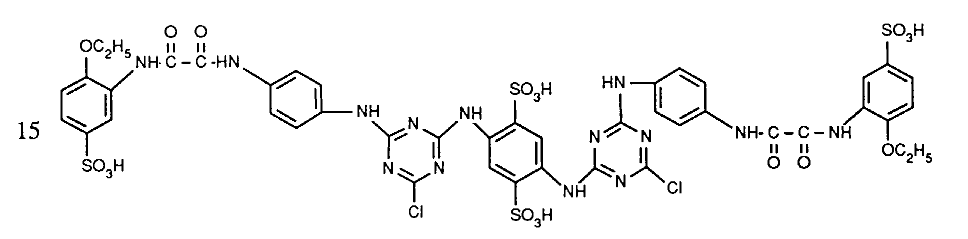

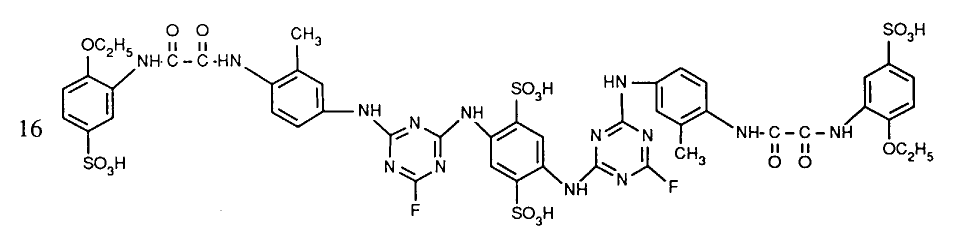

Beispiel 17: Zu einer Dispersion von 2,4 Teilen Cyanurchlorid in 10 Teilen Wasser und 15 Teilen Eis wird eine neutralisierte Lösung von 6,1 Teilen der Verbindung der Formel (16) gemäss Beispiel (9) in 30 Teilen Wasser zugetropft, wobei der pH-Wert durch gleichzeitiges Zutropfen von verdünnter Natriumhydroxidlösung konstant bei ca. 4,5 gehalten wird. Nach beendeter Kondensation versetzt man mit 0,4 Teilen Ethylendiamin, erwärmt auf 30°C und hält den pH-Wert durch gleichzeitiges Zutropfen von verdünnter Natriumhydroxidlösung konstant bei ca. 8,5. Nach Beendigung der zweiten Kondensation wird klärfiltriert und anschliessend das Produkt der Formel 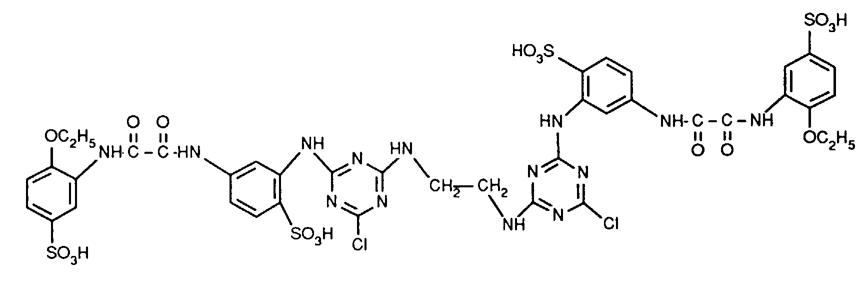 durch Aussalzen mit Kochsalz isoliert.

Beispiele 18-19: Analog wie im Beispiel 17 beschrieben lassen sich die folgenden Verbindungen herstellen:

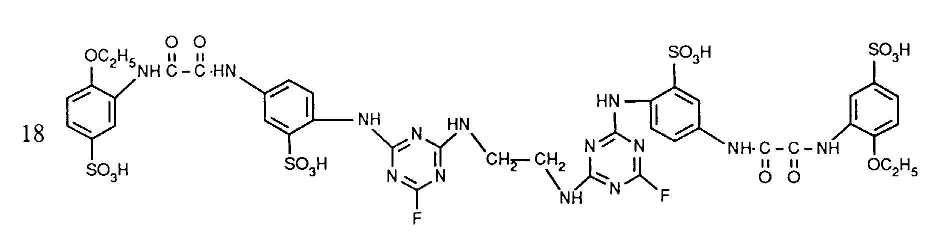

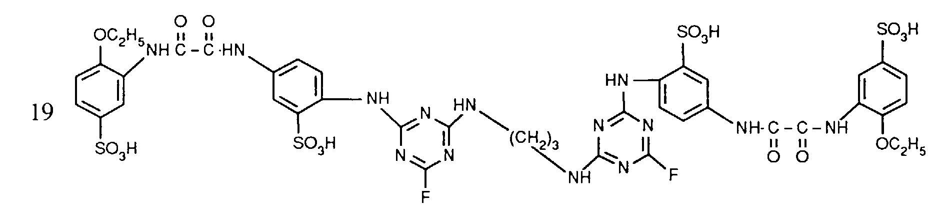

Beispiele 20-22: Analog wie im Beispiel 8 beschrieben lassen sich die folgenden Verbindungen herstellen.

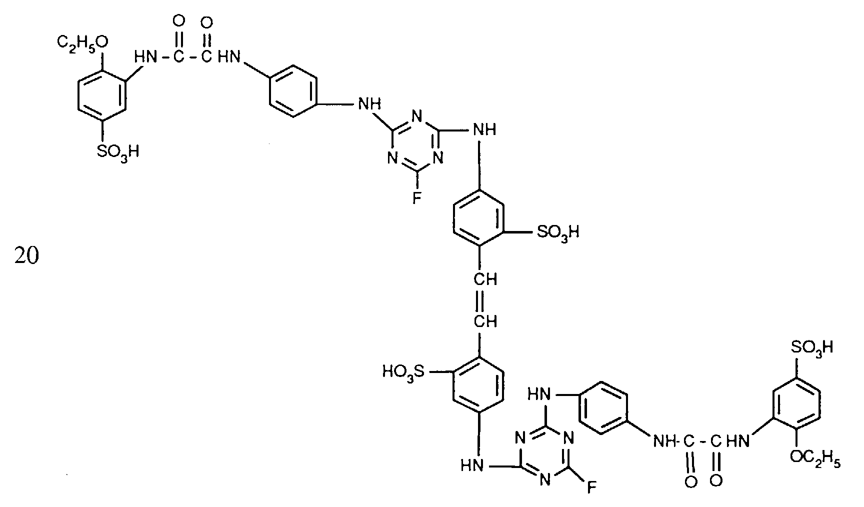

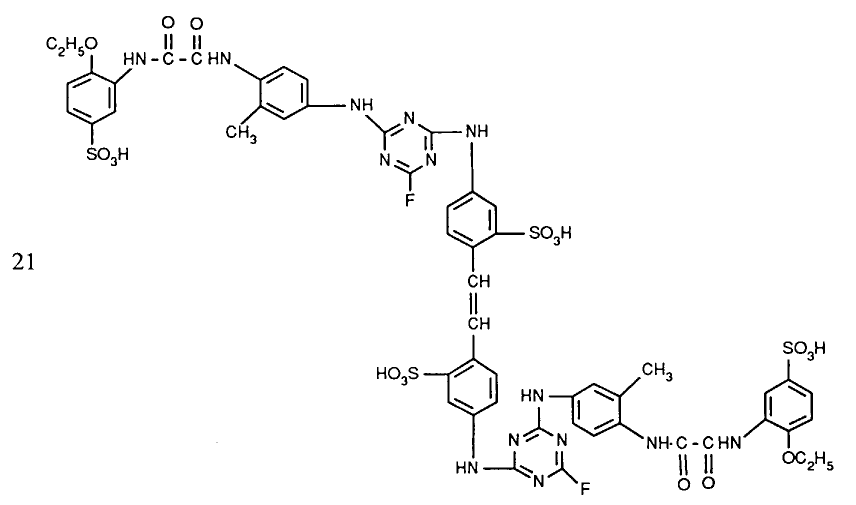

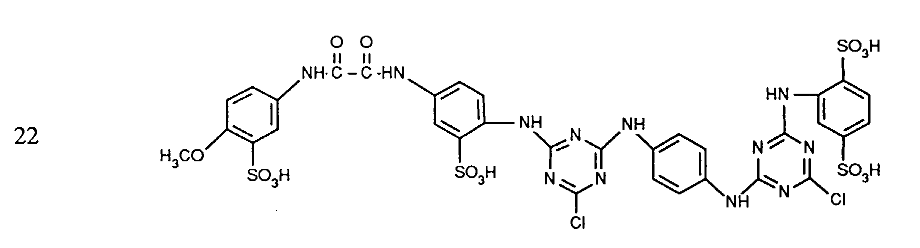

Applikationsbeispiel: In einem Jet-Färbeapparat werden 100 g eines gebleichten Baumwolltrikots 20 Minuten bei 60°C mit einer Flotte enthaltend 1 g der Verbindung gemäss Beispiel 8 und 75 g Natriumsulfat bei einem Flottenverhältnis von 1:15 behandelt. Nach Zugabe von 30 g Natriumcarbonat wird das Baumwolltrikot weitere 60 Minuten bei 60°C behandelt. Dann wird das Fasermaterial der Flotte entnommen, mehrmals mit kaltem, warmen und heissem Wasser gewaschen und getrocknet. Man erhält ein Baumwolltrikot mit einem ausgezeichneten Sonnenschutzfaktor.

## Patentansprüche

1. Verbindungen der Formel 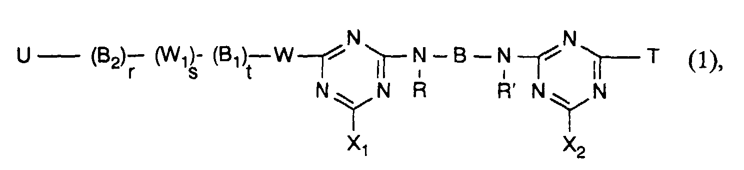 worin
B ein geradkettiges oder verzweigtes und gegebenenfalls durch Hydroxy, Sulfo oder Sulfato substituiertes und/oder durch -O- unterbrochenes C₂-C₁₂-Alkylen, unsubstituiertes oder durch Sulfo, Carboxy, C₁-C₄-Alkyl, C₁-C₄-Alkoxy oder Halogen substituiertes 1,2- 1,3- oder 1,4-Phenylen, gegebenenfalls durch Sulfo substituiertes Naphthylen, Rest der Formel 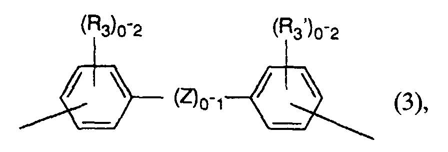 worin Z die Bedeutung -CO-, -NHCO-, -NHCONH-, -(CH₂)₁₋₄-, -NH-, -CH=CH-, -O-, -SO₂- oder -N=N- hat und (R₃)₀₋₂ und (R₃')₀₋₂ unabhängig voneinander jeweils für 0 bis 2 gleiche oder verschiedene Reste aus der Gruppe Sulfo, Methyl, Methoxy und Chlor stehen, unsubstituiertes oder im Phenylenteil durch Sulfo, Methyl,Methoxy, Carboxy oder Chlor substituiertes Phenyl-C₁-C₄-Alkylen, Cyclohexylen, Rest der Formel 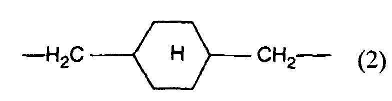 ist, oder zusammen mit -NR- und -NR'- einen heterocyclischen Ring bildet,
B₁ und B₂ unabhängig voneinander je ein geradkettiges oder verzweigtes C₁-C₁₂-Alkylen bedeuten,
R, R' und R₂ unabhängig voneinander je Wasserstoff oder gegebenenfalls durch Halogen, Hydroxy, Cyano, C₁-C₄-Alkoxy C₁-C₄-Alkoxycarbonyl, Carboxy, Sulfamoyl, Sulfo oder Sulfato substituiertes C₁-C₄-Alkyl bedeuten,
U der Rest eines 2-(2'-Hydroxyphenyl)-benztriazol-UV-Absorbers der Formel 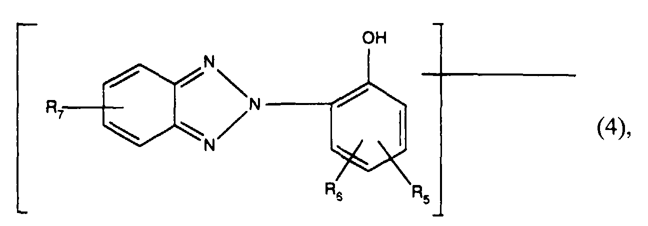 worin
R₅ Wasserstoff, C₁-C₁₂-Alkyl, Phenyl-C₁-C₄-alkyl, C₅-C₈-Cycloalkyl oder ein Rest der Formel 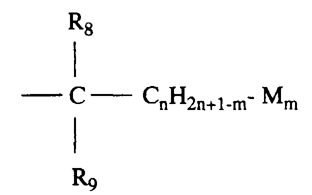 ist, worin R₈ und R₉
unabhängig voneinander Alkyl mit je 1 bis 5 Kohlenstoffatomen sind, oder R₈ zusammen mit dem Rest CₙH₂ₙ₊₁₋ₘ einen Cycloalkylrest mit 5 bis 12 Kohlenstoffatomen bildet, m 1 oder 2, n eine ganze Zahl von 2 bis 20 und M ein Rest der Formel -COOR₁₀ ist, worin R₁₀ Wasserstoff, Alkyl mit 1 bis 12 Kohlenstoffatomen, Alkoxyalkyl mit je 1 bis 20 Kohlenstoffatomen im Alkyl- und Alkoxyteil oder Phenylalkyl mit 1 bis 4 Kohlenstoffatomen im Alkylteil ist,
R₆ Wasserstoff, Sulfo, Halogen, Alkyl mit 1 bis 18 Kohlenstoffatomen, Phenylalkyl mit 1 bis 4 Kohlenstoffatomen im Alkylteil, und
R₇ Wasserstoff, Sulfo, Chlor, Alkyl oder Alkoxy mit je 1 bis 4 Kohlenstoffatomen oder -COOR₁₀, worin R₁₀ die angegebene Bedeutung hat, bedeutet, wobei mindestens einer der Reste R₅ und R₆ von Wasserstoff verschieden ist,
eines 2-Hydroxybenzophenon-UV-Absorbers der Formel, 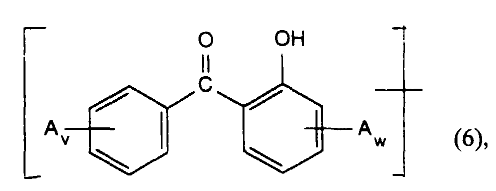 worin v eine ganze Zahl von 1 bis 3 und w 1 oder 2 ist, und die Substituenten A unabhängig voneinander Wasserstoff, Halogen, Hydroxyl, Sulfo, Alkoxy mit 1 bis 12 Kohlenstoffatomen oder Phenyl-C₁-C₄-Alkoxy sind,
eines 2-Hydroxyphenyl,1,3,5-triazin-UV-Absorbers der Formel 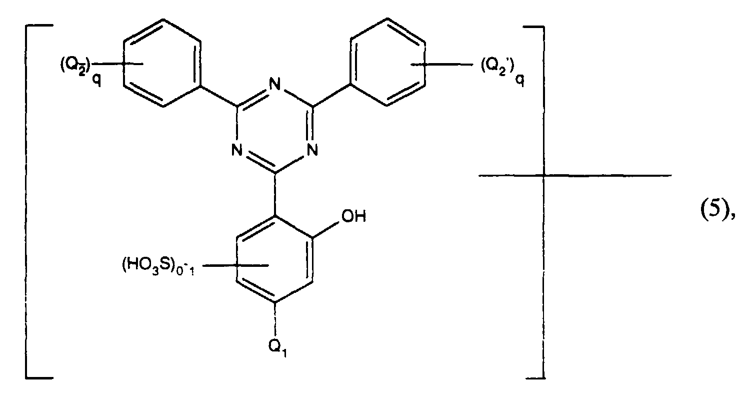 worin q eine ganze Zahl von 1 bis 3 bedeutet und Q₁, Q₂ und Q₂' unabhängig voneinander je Wasserstoff, Hydroxyl, Alkyl mit 1 bis 12 Kohlenstoffatomen, Alkoxy mit 1 bis 18 Kohlenstoffatomen oder gegebenenfalls durch Hydroxy substituiertes C₁-C₄-Alkoxy-C₁-C₄-Alkoxy bedeuten,
eines Oxalsäurediamid-UV-Absorbers der Formel 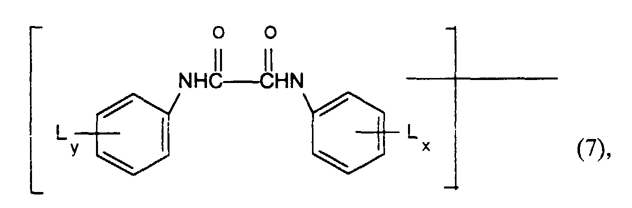 worin x und y unabhängig voneinander je eine ganze Zahl von 0 bis 3 bedeuten, wobei die Summe von (x + y) ≥ 1 ist, und die Substituenten L unabhängig voneinander je Sulfo, unsubstituiertes oder im Alkylteil durch Sulfo substituiertes Alkyl, Alkoxy oder Alkylthio mit je 1 bis 22 Kohlenstoffatomen oder unsubstituiertes oder im Phenylring durch Sulfo substituiertes Phenoxy oder Phenylthio bedeuten,
eines Acrylat-UV-Absorbers oder der Rest einer gegebenenfalls durch Hydroxy oder C₁-C₄-Alkyl substituierten Benzoesäure oder deren Phenyl-, C₁-C₈-Alkylphenyl- oder C₁-C₁₈-Alkylester ist,
W für -NR₂-, -O- oder -S- steht,
W₁ ein Rest -C(O)O-, -O(O)C-, -C(O)NH- oder -HN(O)C- ist,
X₁ und X₂ unabhängig voneinander je Halogen, Hydroxy, -NH₂, unsubstituiertes oder im Alkylteil durch Hydroxy, Carboxy, Sulfo, Sulfato oder C₁-C₄-Alkoxy substituiertes N-Mono- oder N,N-Di-C₁-C₄-Alkylamino, Cyclohexylamino, unsubstituiertes oder im Phenylteil durch C₁-C₄-Alkyl, C₁-C₄-Alkoxy, Carboxy, Sulfo, Halogen oder durch einen Rest der Formel
-SO₂-Y (8a),
-CONH-(CH₂)ₚ-SO₂-Y (8b),
worin Y Vinyl oder einen Rest -CH₂-CH₂-G bedeutet, G eine Abgangsgruppe ist und p eine ganze Zahl 1 bis 6 bedeutet, substituiertes Phenylamino, N-C₁-C₄-Alkyl-N-phenylamino, 3-Carboxypyridin-1-yl oder 3-Carbamoylpyridin-1-yl bedeuten,
T unabhängig eine der für X₁ angegebenen Bedeutungen hat oder für einen C₁-C₄-Alkoxyrest, einen gegebenenfalls durch C₁-C₄-Alkyl, C₁-C₄-Alkoxy, Halogen, Hydroxy, Carboxy oder Sulfo substituierten Phenoxyrest, einen C₁-C₄-Alkylthiorest oder einen gegebenenfalls durch C₁-C₄-Alkyl, C₁-C₄-Alkoxy, Halogen, Hydroxy, Carboxy oder Sulfo substituierten Phenylthiorest oder für einen Piperidino-, Piperazino- oder Morpholinorest steht oder unabhängig ein Rest U-(B₂)ᵣ-(W₁)ₛ-(B₁)ₜ-W-, worin U, B₁, B₂, W und W₁ jeweils die oben angegebene Bedeutung haben, ist, und
r, s und t unabhängig voneinander je die Zahl 0 oder 1 bedeuten, wobei s 0 bedeutet, wenn t 0 ist,
mit der Massgabe, dass die Verbindungen der Formel (1) mindestens eine Sulfo- oder Sulfatogruppe und mindestens eine alkalisch abspaltbare Gruppe aufweisen.

2. Verbindungen gemäss Anspruch 1, dadurch gekennzeichnet, dass r und s jeweils die Zahl 0 bedeuten.

3. Verbindungen gemäss Anspruch 1 oder 2, dadurch gekennzeichnet, dass B unsubstituiertes oder durch Hydroxy, Sulfo oder Sulfato substituiertes C₂-C₆-Alkylen, unsubstituiertes oder durch Sulfo, Carboxy, Chlor, Methyl oder Methoxy substituiertes 1,3- oder 1,4-Phenylen, durch 1 oder 2 Sulfogruppen substituiertes Naphthylen oder einen Rest der Formel 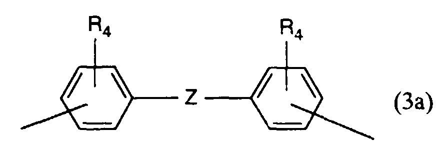 oder 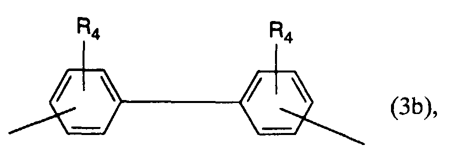 worin Z -NHCONH-, -O-, -NH-, -CH=CH- oder -CH₂- und R₄ Wasserstoff oder Sulfo sind, bedeutet.

4. Verbindungen gemäss einem der Ansprüche 1 bis 3, dadurch gekennzeichnet, dass B für 4-Sulfophenylen-1,3, 3-Sulfophenylen-1,4, 3,6-Disulfophenylen-1,4 oder 4,6-Disulfophenylen-1,3 steht.

5. Verbindungen gemäss einem der Ansprüche 1 bis 4, dadurch gekennzeichnet, dass B₁ geradkettiges oder verzweigtes C₁-C₆-Alkylen ist.

6. Verbindungen gemäss einem der Ansprüche 1 bis 5, dadurch gekennzeichnet, dass U
(i) einen Rest der Formel 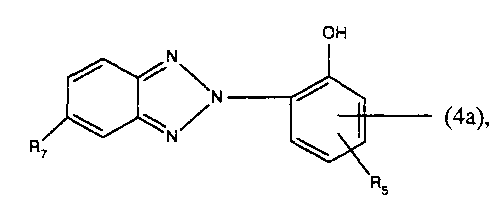 worin R₅ Wasserstoff, C₁-C₄-Alkyl oder Sulfo und R₇ Wasserstoff, Sulfo oder Carboxy bedeuten, und worin einer der Reste R₅ und R₇ verschieden von Wasserstoff ist;
(ii) einen Rest der Formel 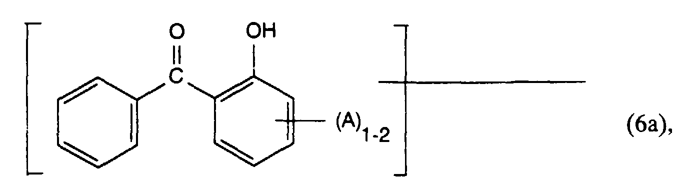 worin (A)₁₋₂ für 1 oder 2 gleiche oder verschiedene Reste aus der Gruppe C₁-C₁₂-Alkoxy und Sulfo steht;
(iii) einen Rest der Formel 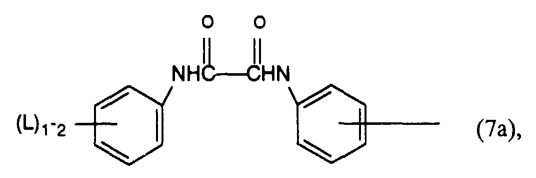 worin (L)₁₋₂ für 1 oder 2 Reste L aus der Gruppe Sulfo, C₁-C₄-Alkyl und C₁-C₁₂-Alkoxy steht; oder
(iv) einen unsubstituierten oder durch Hydroxy oder C₁-C₄-Alkyl substituierten Benzoesäurerest oder dessen Phenyl-, C₁-C₈-Alkylphenyl- oder C₁-C₁₈-Alkylester bedeutet.

7. Verbindungen gemäss einem der Ansprüche 1 bis 6, dadurch gekennzeichnet, dass R und R' unabhängig voneinander je Wasserstoff oder C₁-C₄-Alkyl bedeuten und X₁ und X₂ je für Chlor oder Fluor stehen.

8. Verbindungen gemäss einem der Ansprüche 1 bis 7, dadurch gekennzeichnet, dass W ein Rest -NR₂- ist und R₂ Wasserstoff oder C₁-C₄-Alkyl bedeutet.

9. Verbindungen gemäss einem der Ansprüche 1 bis 8, dadurch gekennzeichnet, dass T für -NH₂, unsubstituiertes oder im Alkylteil durch Hydroxy, Carboxy, Sulfo, Sulfato oder C₁-C₄-Alkoxy substituiertes N-Mono- oder N,N-Di-C₁-C₄-Alkylamino, Cyclohexylamino, unsubstituiertes oder im Phenylteil durch C₁-C₄-Alkyl, C₁-C₄-Alkoxy, Carboxy, Sulfo, Halogen oder durch einen Rest der Formel
-SO₂-Y (8a),
-CONH-(CH₂)ₚ-SO₂-Y (8b),
worin Y Vinyl oder einen Rest -CH₂-CH₂-G bedeutet, G eine Abgangsgruppe ist und p eine ganze Zahl 1 bis 6 bedeutet, substituiertes Phenylamino, N-C₁-C₄-Alkyl-N-phenylamino, Morpholino, C₁-C₄-Alkoxy steht oder einen Rest -W-(B₁)ₜ-(W₁)ₛ-(B₂)ᵣ-U bedeutet.

10. Verbindungen gemäss einem der Ansprüche 1 bis 9, dadurch gekennzeichnet, dass T für Amino, Methylamino, Ethylamino, Carboxymethylamino, β-Hydroxyethylamino, β-Sulfoethylamino, N,N-Di-β-hydroxyethylamino, Cyclohexylamino, o-, m- oder p-Methylphenylamino, o-, m- oder p-Methoxyphenylamino, o-, m- oder p-Sulfophenylamino, 2,4- oder 2,5-Disulfophenylamino, o-Carboxyphenylamino, N-Ethyl-N-phenylamino, N-Methyl-N-phenylamino oder für Phenylamino, welches durch einen Rest der Formel
-SO₂-Y (8a)
oder
-CONH-(CH₂)ₚ-SO₂-Y (8b),
worin Y Vinyl oder einen Rest -CH₂-CH₂-G bedeutet, G eine Abgangsgruppe ist und p eine ganze Zahl 1 bis 6 bedeutet, substituiert ist, steht.

11. Verbindungen der Formel (1) gemäss einem der Ansprüche 1 bis 9, dadurch gekennzeichnet, dass T für einen Rest -W-(B₁)ₜ-U steht und r und s je die Zahl 0 bedeuten.

12. Verbindungen gemäss Anspruch 1 der Formel 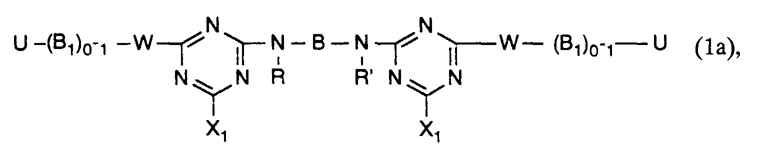 worin B 4-Sulfophenylen-1,3, 3-Sulfophenylen-1,4, 3,6-Disulfophenylen-1,4 oder 4,6-Disulfophenylen-1,3 bedeutet, B₁ geradkettiges oder verzweigtes C₁-C₆-Alkylen ist, R und R' unabhängig voneinander je Wasserstoff, Methyl oder Ethyl bedeuten, W für die Gruppe -NH- steht, X₁ und X₂ jeweils Chlor oder Fluor bedeuten, und U
(i) einen Rest der Formel 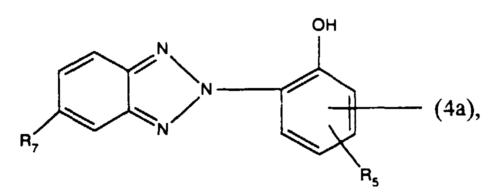 worin R₅ Wasserstoff, C₁-C₄-Alkyl oder Sulfo und R₇ Wasserstoff, Sulfo oder Carboxy bedeuten, und worin einer der Reste R₅ und R₇ verschieden von Wasserstoff ist;
(ii) einen Rest der Formel 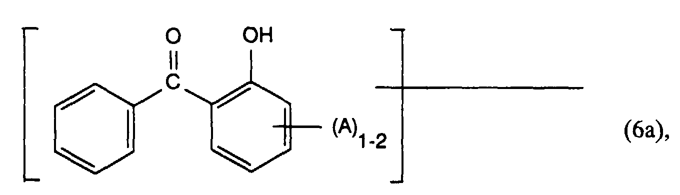 worin (A)₁₋₂ für 1 oder 2 gleiche oder verschiedene Reste aus der Gruppe C₁-C₁₂-Alkoxy und Sulfo steht;
(iii) einen Rest der Formel 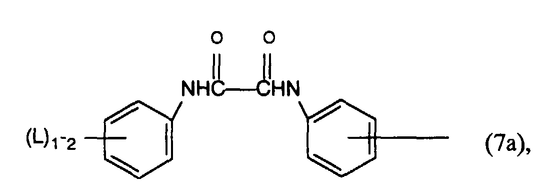 worin (L)₁₋₂ für 1 oder 2 Reste L aus der Gruppe Sulfo, C₁-C₄-Alkyl und C₁-C₁₂-Alkoxy steht; oder
(iv) einen unsubstituierten oder durch Hydroxy oder C₁-C₄-Alkyl substituierten Benzoesäurerest oder dessen Phenyl-, C₁-C₈-Alkylphenyl- oder C₁-C₁₈-Alkylester bedeutet.

13. Verbindungen der Formel (1a) gemäss Anspruch 12, dadurch gekennzeichnet, dass U ein Rest der Formel (7a) ist.

14. Verfahren zur Herstellung von Verbindungen der Formel (1) gemäss Anspruch 1, dadurch gekennzeichnet, dass man eine Verbindung der Formel
U - (B₂)ᵣ - (W₁)ₛ - (B₁)ₜ- W - H (8),
eine Verbindung der Formel
H - RN - B - NR'- H (9),
eine Verbindung der Formel 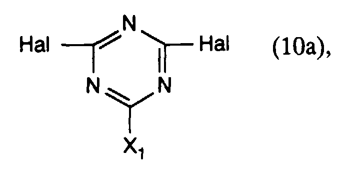 eine Verbindung der Formel 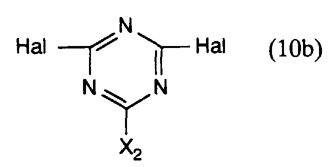 und
gegebenenfalls eine Verbindung der Formel
T* - H (11),
worin U, B, B₁, B₂, W, W₁, R, R', X₁, X₂, r, s und t jeweils die im Anspruch 1 angegebene Bedeutung haben, Hal Halogen, vorzugsweise Fluor oder Chlor, bedeutet und T* die zuvor für T angegebene Bedeutung ausgenommen Halogen hat, in beliebiger Reihenfolge miteinander umsetzt.

15. Verfahren zur Erhöhung des Sonnenschutzfaktors von textilen Fasermaterialien, dadurch gekennzeichnet, dass man eine oder mehrere Verbindungen der Formel (1) gemäss Anspruch 1 in einer wässrigen oder wässrig-organischen Lösung auf die Fasermaterialien aufbringt und anschliessend fixiert.

16. Verwendung von Verbindungen der Formel (1) gemäss Anspruch 1 zur Erhöhung des Lichtschutzfaktors von ungefärbten, gefärbten oder bedruckten textilen Fasermaterialien.

17. Verwendung von Verbindungen der Formel (1) gemäss Anspruch 1 zur photochemischen Stabilisierung von ungefärbten, gefärbten oder bedruckten textilen Fasermaterialien.

## Claims

1. A compound of formula 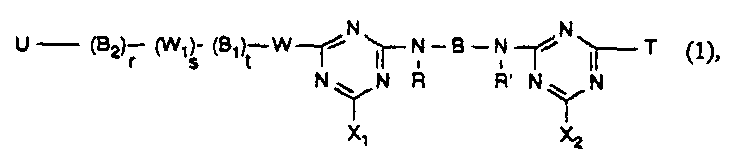 wherein
B is a straight-chain or branched C₂-C₁₂alkylene which is unsubstituted or substituted by hydroxy, sulfo or sulfato and/or uninterrupted or interrupted by -O-, or is unsubstituted or sulfo-, carboxy-, C₁-C₄alkyl-, C₁-C₄-alkoxy- or halo-substituted 1,2-, 1,3- or 1,4-phenylene, or is unsubstituted or sulfo-substituted naphthylene, or is a radical of the formula 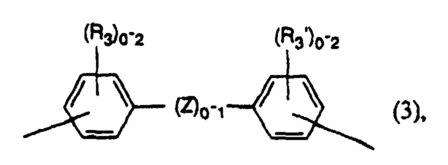 in which Z has the meaning -CO-, -NHCO-, -NHCONH-, -(CH₂)₁₋₄-, -NH-, -CH=CH-, -O-, -SO₂- or -N=N- and (R₃)₀₋₂ and (R₃')₀₋₂ independently of one another are in each case from 0 to 2 identical or different radicals selected from the group consisting of sulfo, methyl, methoxy and chloro, or is phenyl-C₁-C₄alkylene which is unsubstituted or is substituted in the phenyl moiety by sulfo, methyl, methoxy, carboxy or chloro, or is cyclohexylene, or is a radical of the formula 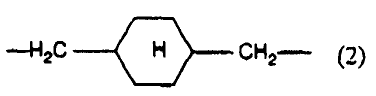 or together with -NR- and -NR'- forms a heterocyclic ring,
B₁ and B₂ independently of one another are each a straight-chain or branched C₁-C₁₂alkylene,
R, R' and R₂ independently of one another are each hydrogen or are C₁-C₄alkyl which is unsubstituted or substituted by halo, hydroxyl, cyano, C₁-C₄alkoxy, C₁-C₄-alkoxycarbonyl, carboxy, sulfamoyl, sulfo or sulfato,
U is the radical of a 2-(2'-hydroxyphenyl)benzotriazole UV absorber of the formula 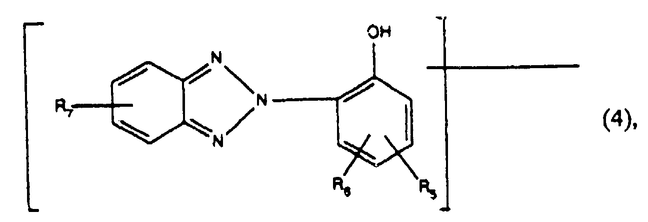 in which
R₅ is hydrogen, C₁-C₁₂alkyl, phenyl-C₁-C₄alkyl, C₅-C₈-cycloalkyl or a radical of the formula 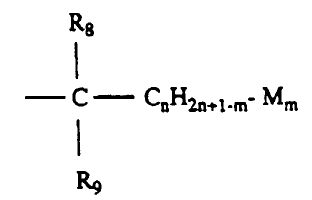 in which R₈ and R₉ independently of one another are alkyl having in each case 1 to 5 carbon atoms, or R₈ together with the radical CₙR₂ₙ₊₁₋ₘ forms a cycloalkyl radical having 5 to 12 carbon atoms, m is 1 or 2, n is an integer from 2 to 20 and M is a radical of the formula -COOR₁₀, in which
R₁₀ is hydrogen, alkyl of 1 to 12 carbon atoms, alkoxyalkyl having in each case 1 to 20 carbon atoms in the alkyl and alkoxy moieties or phenylalkyl having 1 to 4 carbon atoms in the alkyl moiety,
R₆ is hydrogen, sulfo, halo, alkyl of 1 to 18 carbon atoms, phenylalkyl having 1 to 4 carbon atoms in the alkyl moiety, and
R₇ is hydrogen, sulfo, chloro, alkyl or alkoxy having in each case 1 to 4 carbon atoms or is -COOR₁₀, in which R₁₀ is as defined, at least one of the radicals R₅ and R₆ being other than hydrogen,
of a 2-hydroxybenzophenone UV absorber of the formula 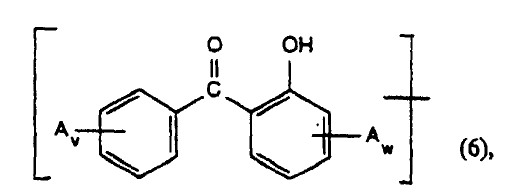 in which v is an integer from 1 to 3 and w is 1 or 2, and the substituents A independently of one another are hydrogen, halo, hydroxyl, sulfo, alkoxy of 1 to 12 carbon atoms or phenyl-C₁-C₄alkoxy,
of a 2-hydroxyphenyl-1,3,5-triazine UV absorber of the formula 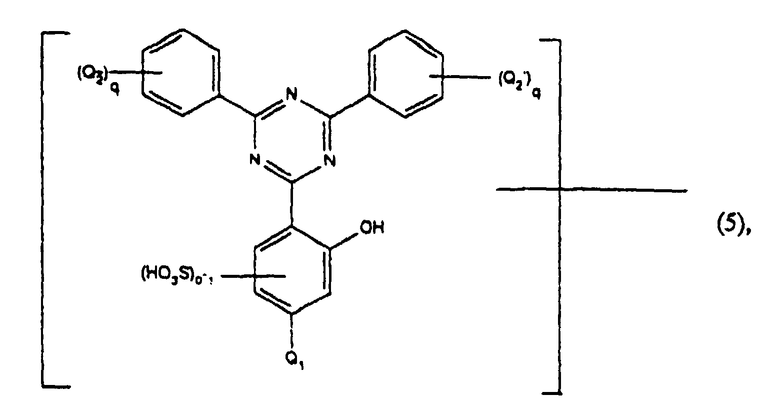 in which q is an integer from 1 to 3 and Q₁, Q₂ and Q₂' independently of one another are each hydrogen, hydroxy, alkyl of 1 to 12 carbon atoms, alkoxy of 1 to 18 carbon atoms or unsubstituted or hydroxy-substituted C₁-C₄alkoxy-C₁-C₄alkoxy,
of an oxalamide UV absorber of the formula 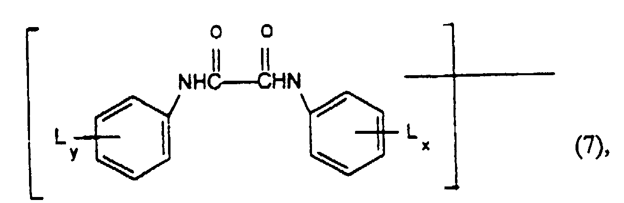 in which x and y independently of one another are each an integer from 0 to 3, the sum (x + y) being ≥ 1, and the substituents L independently of one another are each sulfo, alkyl, alkoxy or alkylthio each of 1 to 22 carbon atoms and unsubstituted or substituted in the alkyl moiety by sulfo, or phenoxy or phenylthio each unsubstituted or substituted in the phenyl ring by sulfo,
of an acrylate UV absorber, or the radical of an unsubstituted or hydroxy- or C₁-C₄alkyl-substituted benzoic acid or a phenyl, C₁-C₈alkylphenyl or C₁-C₁₈alkyl ester thereof,
W is -NR₂-, -O- or -S-,
W₁ is a radical -C(O)O-, -O(O)C-, -C(O)NH- or -HN(O)C-,
X₁ and X₂ independently of one another are each halo, hydroxy, -NH₂, N-mono- or N,N-di-C₁-C₄alkylamino which is unsubstituted or is substituted in the alkyl moiety by hydroxy, carboxy, sulfo, sulfato or C₁-C₄alkoxy, or are cyclohexylamino, or are phenylamino which is unsubstituted or is substituted in the phenyl moiety by C₁-C₄alkyl, C₁-C₄alkoxy, carboxy, sulfo, halo or by a radical of the formula
-SO₂-Y (8a),
-CONH-(CH₂)ₚ-SO₂-Y (8b),
in which Y is vinyl or a radical -CH₂-CH₂-G, G is a leaving group and p is an integer from 1 to 6,
or are N-C₁-C₄alkyl-N-phenylamino, 3-carboxypyridin-1-yl or 3-carbamoylpyridin-1-yl,
T independently has one of the meanings indicated for X₁ or is a C₁-C₄alkoxy radical, an unsubstituted or C₁-C₄alkyl, C₁-C₄alkoxy-, halo-, hydroxy-, carboxy- or sulfo-substituted phenoxy radical, a C₁-C₄alkylthio radical or an unsubstituted or C₁-C₄alkyl-, C₁-C₄alkoxy-, halo-, hydroxy-, carboxy- or sulfo-substituted phenylthio radical or is a piperidino, piperazino or morpholino radical or independently is a radical U-(B₂)ᵣ-(W₁)ₛ-(B₁)ₜ-W-, in which U, B₁, B₂, W and W₁ are each as defined above, and
r, s and t are each independently of one another 0 or 1, and s is 0 when t is 0,
with the proviso that the compound of formula (1) contains at least one sulfo or sulfato group and at least one group which is removable under alkaline conditions.

2. A compound according to claim 1, wherein r and s are each 0.

3. A compound according to claim 1 or claim 2, wherein B is C₂-C₆alkylene which is unsubstituted or substituted by hydroxy, sulfo or sulfato, or is 1,3- or 1,4-phenylene, each unsubstituted or substituted by sulfo, carboxy, chloro, methyl or methoxy, or is naphthylene which is substituted by 1 or 2 sulfo groups, or is a radical of formula 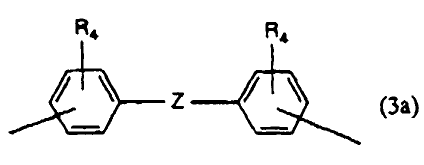 or 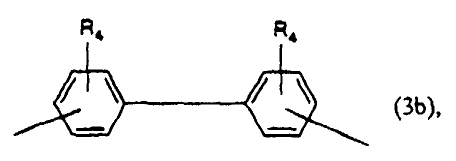 in which Z is -NHCONH-, -O-, -NH-, -CH=CH- or -CH₂- and R₄ is hydrogen or sulfo.

4. A compound according to any one of claims 1 to 3, wherein B is 4-sulfo-1,3-phenylene, 3-sulfo-1,4-phenylene, 3,6-disulfo-1,4-phenylene or 4,6-disulfo-1,3-phenylene.

5. A compound according to any one of claims 1 to 4, wherein B₁ is straight-chain or branched C₁-C₆-alkylene.

6. A compound according to any one of claims 1 to 5, wherein U is
(i) a radical of formula 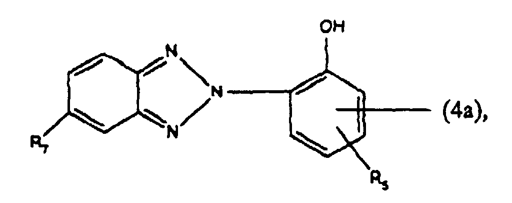 in which R₅ is hydrogen, C₁-C₄alkyl or sulfo and R₇ is hydrogen, sulfo or carboxy, and in which one of R₅ and R₇ is different from hydrogen;
(ii) a radical of formula 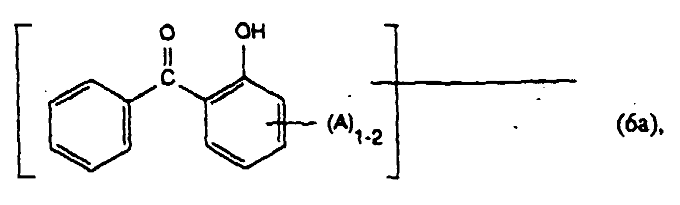 in which (A)₁₋₂ is 1 or 2 identical or different radicals selected from the group consisting of C₁-C₁₂-alkoxy and sulfo;
(iii) a radical of formula 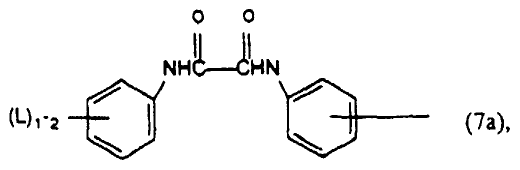 in which (L)₁₋₂ is 1 or 2 radicals L selected from the group consisting of sulfo, C₁-C₄alkyl and C₁-C₁₂alkoxy; or
(iv) an unsubstituted or hydroxy- or C₁-C₄alkyl-substituted benzoic acid radical or a phenyl, C₁-C₈-alkylphenyl or C₁-C₁₈alkyl ester thereof.

7. A compound according to any one of claims 1 to 6, wherein R and R' are each independently of the other hydrogen or C₁-C₄alkyl and X₁ and X₂ are each chloro or fluoro.

8. A compound according to any one of claims 1 to 7, wherein W is a radical -NR₂- and R₂ is hydrogen or C₁-C₄alkyl.

9. A compound according to any one of claims 1 to 8, wherein T is -NH₂, N-mono- or N,N-di-C₁-C₄alkylamino which is unsubstituted or substituted in the alkyl moiety by hydroxy, carboxy, sulfo, sulfato or C₁-C₄-alkoxy, or is cyclohexylamino, or is phenylamino which is unsubstituted or is substituted in the phenyl moiety by C₁-C₄alkyl, C₁-C₄alkoxy, carboxy, sulfo, halo or by a radical of the formula
-SO₂-Y (8a),
-CONH-(CH₂)ₚ-SO₂-Y (8b),
in which Y is vinyl or a radical -CH₂-CH₂-G, G is a leaving group and p is an integer from 1 to 6,
or is N-C₁-C₄alkyl-N-phenylamino, morpholino or C₁-C₄-alkoxy or is a radical -W-(B₁)ₜ-(W₁)ₛ-(B₂)ᵣ-U.

10. A compound according to any one of claims 1 to 9, wherein T is amino, methylamino, ethylamino, carboxymethylamino, β-hydroxyethylamino, β-sulfoethylamino, N,N-di-β-hydroxyethylamino, cyclohexylamino, o-, m- or p-methylphenylamino, o-, m- or p-methoxyphenylamino, o-, m- or p-sulfophenylamino, 2,4- or 2,5-disulfophenylamino, o-carboxyphenylamino, N-ethyl-N-phenylamino, N-methyl-N-phenylamino, or phenylamino which is substituted by a radical of formula
-SO₂-Y (8a)
or
-CONH-(CH₂)ₚ-SO₂-Y (8b),
in which Y is vinyl or a radical -CH₂-CH₂-G, G is a leaving group and p is an integer from 1 to 6.

11. A compound of the formula (1) according to any one of claims 1 to 9, wherein T is a radical -W-(B₁)ₜ-U and r and s are each 0.

12. A compound according to claim 1 of formula 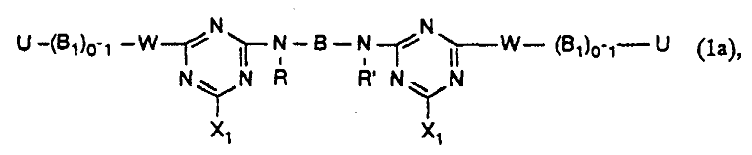 wherein B is 4-sulfo-1,3-phenylene, 3-sulfo-1,4-phenylene, 3,6-disulfo-1,4-phenylene or 4,6-disulfo-1,3-phenylene, B₁ is straight-chain or branched C₁-C₆-alkylene, R and R' are each independently of the other hydrogen, methyl or ethyl, W is the -NH- group, X₁ and X₂ are each chloro or fluoro, and U is
(i) a radical of formula 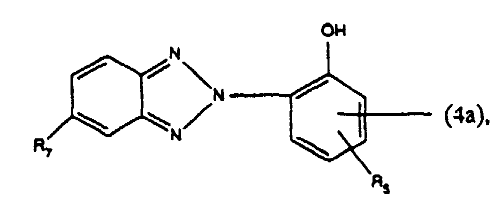 in which R₅ is hydrogen, C₁-C₄alkyl or sulfo and R₇ is hydrogen, sulfo or carboxy, and in which one of R₅ and R₇ is other than hydrogen;
(ii) a radical of formula 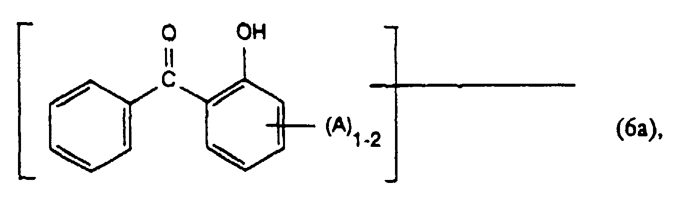 in which (A)₁₋₂ is 1 or 2 identical or different radicals selected from the group consisting of C₁-C₁₂-alkoxy and sulfo;
(iii) a radical of formula 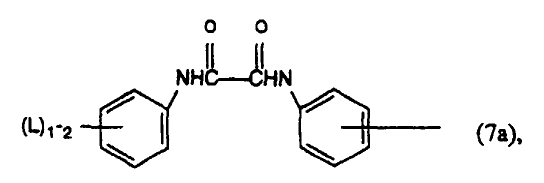 in which (L)₁₋₂ is 1 or 2 radicals L selected from the group consisting of sulfo, C₁-C₄alkyl and C₁-C₁₂alkoxy; or
(iv) an unsubstituted or hydroxy- or C₁-C₄alkyl-substituted benzoic acid radical or a phenyl, C₁-C₈-alkylphenyl or C₁-C₁₈alkyl ester thereof.

13. A compound of formula (1a) according to claim 12, wherein U is a radical of formula (7a).

14. A process for the preparation of a compound of formula (1) according to claim 1, which comprises reacting a compound of formula
U-(B₂)ᵣ-(W₁)ₛ-(B₁)ₜ-W-H (8)
a compound of formula
H - RN - B - NR' - H (9)
a compound of formula 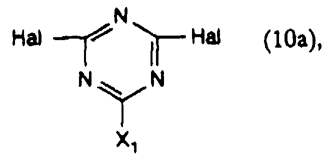 a compound of formula 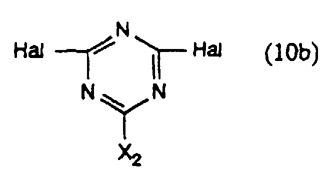 and
optionally, a compound of formula
T* - H (11)
wherein U, B, B₁, B₂, W, W₁, R, R', X₁, X₂, r, s and t are each as defined in claim 1, Hal is halo, preferably fluoro or chloro, and T* has the meaning previously given for T, except halo, with one another in any order.

15. A process for enhancing the sun protection factor of textile fibre materials, which comprises applying one or more than one compound of formula (1) according to claim 1, in an aqueous or aqueous-organic solution, to said materials and subsequently fixing said compound or compounds thereon.

16. The use of a compound of formula (1) according to claim 1 for enhancing the sun protection factor of undyed, dyed or printed textile fibre materials.

17. The use of a compound of formula (1) according to claim 1 for the photochemical stabilization of undyed, dyed or printed textile fibre materials.

## Revendications

1. Composé de formule 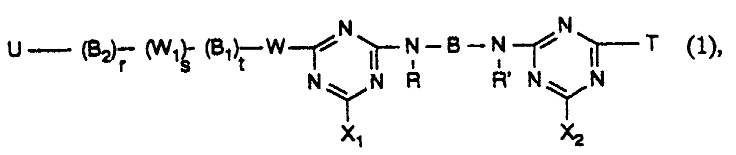 dans laquelle
B représente un groupe alkylène en C₂ à C₁₂ à chaîne linéaire ou ramifiée et éventuellement substitué par un groupe hydroxy, sulfo ou sulfate et/ou interrompu par -O-, un groupe 1,2-, 1,3- ou 1,4-phénylène non substitué ou substitué par un groupe sulfo, carboxy, alkyle en C₁ à C₄, alcoxy en C₁ à C₄ ou un atome d'halogène, un groupe naphtylène éventuellement substitué par un groupe sulfo, le groupe de formule 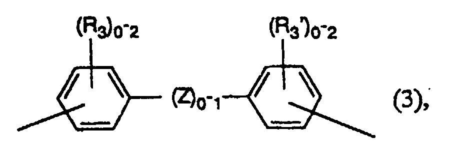 dans laquelle
Z représente -CO-, -NHCO-, -NHCONH-, -(CH₂)₁₋₄-, -NH-, -CH=CH-, -O-, -SO₂- ou -N=N- et (R₃)₀₋₂ et (R₃')₀₋₂ représentent respectivement indépendamment l'un de l'autre de 0 à 2 groupes identiques ou différents choisis parmi un groupe sulfo, méthyle, méthoxy et un atome de chlore, phényl-alkylène en C₁ à C₄ non substitué ou substitué dans la partie phényle par un groupe sulfo, méthyle, méthoxy, carboxy ou un atome de chlore, cyclohexylène, un groupe de formule 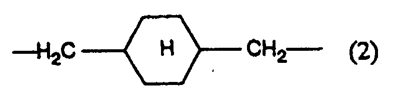 ou forment ensemble avec -NR- et -NR'- un noyau hétérocyclique,
B₁ et B₂ représentent respectivement indépendamment l'un de l'autre un groupe alkylène en C₁ à C₁₂ à chaîne linéaire ou ramifiée,
R, R' et R₂ représentent respectivement indépendamment les uns des autres un atome d'hydrogène ou un groupe alkyle en C₁ à C₄ éventuellement substitué par un atome d'halogène, un groupe hydroxy, cyano, alcoxy en C₁ à C₄, alcoxy en C₁ à C₄-carbonyle, carboxy, sulfamoyle, sulfo ou sulfato,
U représente le résidu d'un absorbant UV 2-(2'-hydroxyphényl)-benzotriazole de formule 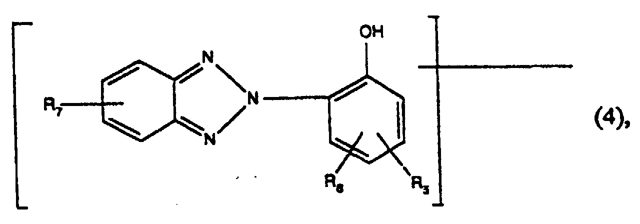 dans laquelle
R₅ représente un atome d'hydrogène, un groupe alkyle en C₁ à C₁₂, phényl-alkyle en C₁ à C₄, cycloalkyle en C₅ à C₈ ou un groupe de formule 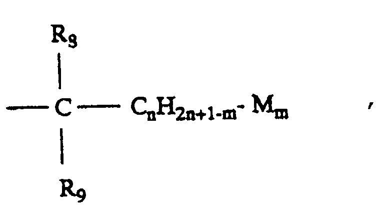 dans laquelle R₈ et R₉ représentent indépendamment l'un de l'autre un groupe alkyle ayant respectivement de 1 à 5 atomes de carbone, ou R₈ forme ensemble avec le groupe CₙH₂ₙ₊₁₋ₘ un groupe cycloalkyle ayant de 5 à 12 atomes de carbone, m est 1 ou 2, n un nombre entier de 2 à 20 et M un groupe de formule -COOR₁₀, dans laquelle
R₁₀ représente un atome d'hydrogène, un groupe alkyle ayant de 1 à 12 atomes de carbone, alcoxyalkyle ayant respectivement de 1 à 20 atomes de carbone dans la partie alkyle et alcoxy ou phénylalkyle ayant de 1 à 4 atomes de carbone dans la partie alkyle,
R₆ représente un atome d'hydrogène, un groupe sulfo, un atome d'halogène, un groupe alkyle ayant de 1 à 18 atomes de carbone, phénylalkyle ayant de 1 à 4 atomes de carbone dans la partie alkyle, et
R₇ représente un atome d'hydrogène, un groupe sulfo, un atome de chlore, un groupe alkyle ou alcoxy ayant de 1 à 4 atomes de carbone ou -COOR₁₀-, dans laquelle R₁₀ a la signification indiquée, au moins un des groupes R₅ et R₆ étant différent de l'hydrogène,
d'un absorbant UV 2-hydroxybenzophénone de formule 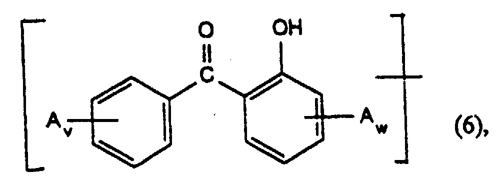 dans laquelle v est un nombre entier de 1 à 3 et w est 1 ou 2, et les substituants A sont indépendamment les uns des autres un atome d'hydrogène, un atome d'halogène, un groupe hydroxyle, sulfo, alcoxy ayant de 1 à 12 atomes de carbone ou phényl-alcoxy en C₁ à C₄,
d'un absorbant UV 2-hydroxyphényl-1,3,5-triazine de formule 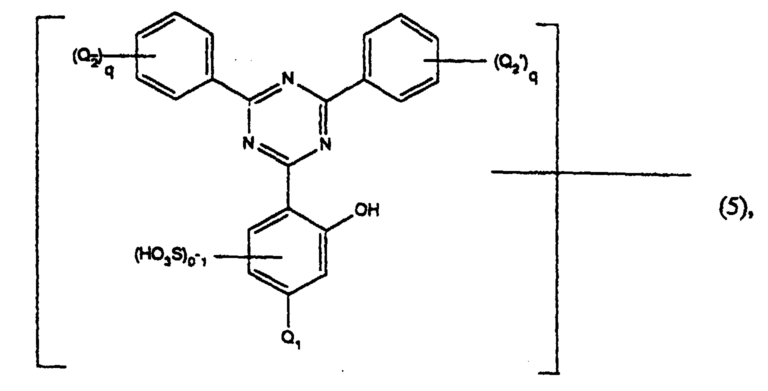 dans laquelle q représente un nombre entier allant de 1 à 3 et Q₁, Q₂ et Q₂' représentent respectivement les uns des autres un atome d'hydrogène un groupe hydroxyle, alkyle ayant de 1 à 12 atomes de carbone, alcoxy ayant de 1 à 18 atomes de carbone ou alcoxy en C₁ à C₄-alcoxy en C₁ à C₄ éventuellement substitué par un groupe hydroxy, d'un absorbant UV oxaldiamide de formule 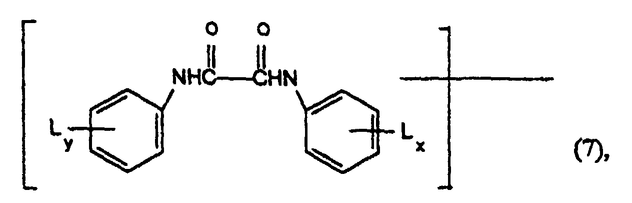 dans laquelle x et y représentent indépendamment l'un de l'autre respectivement un nombre entier allant de 0 à 3, la somme de (x + y) étant ≥ 1 et les substituants L représentent indépendamment les uns des autres respectivement un groupe sulfo, un groupe alkyle, alcoxy ou alkylthio ayant de 1 à 22 atomes de carbone non substitué ou substitué dans la partie alkyle par un groupe sulfo ou un groupe phénoxy ou phénylthio non substitué ou substitué dans le cycle phényle par un groupe phénoxy ou phénylthio substitué par un groupe sulfo,
d'un absorbant UV acrylate ou représente le résidu d'un acide benzoïque éventuellement substitué par un groupe hydroxy ou alkyle en C₁ à C₄ ou son ester phénylique, alkyle en en C₁ à C₈-phénylique ou alkylique en C₁ à C₁₈, W représente -NR₂-, -O- ou -S-,
W₁ représente un groupe -C(O)O-, -O(O)C-, -C(O)NH- ou -NH(O)C-,
X₁ et X₂ représentent indépendamment l'un de l'autre respectivement un atome d'halogène, un groupe hydroxy, -NH₂-, N-Mono- ou N,N-Di-alkylamino en C₁ à C₄ non substitué ou substitué dans la partie alkyle par un groupe hydroxy, carboxy, sulfo, sulfato ou alcoxy en C₁ à C₄, cyclohexylamino, phénylamino non substitué ou substitué dans la partie phényle par un groupe alkyle en C₁ à C₄, alcoxy en C₁ à C₄, carboxy, sulfo, un atome d'halogène ou par un groupe de formule
-SO₂-Y (8a),
-CONH-(CH₂)ₚ-SO₂-Y (8b),
dans lesquelles Y représente un groupe vinyle ou un groupe -CH₂-CH₂-G, G est un groupe partant et p un nombre entier de 1 à 6,
N-alkyle en C₁ à C₄-N-phénylamino, 3-carboxypyridin-1-yle ou 3-carbamoylpyridin-1-yle, X₁ et X₂ représentent de préférence indépendamment l'un de l'autre respectivement un atome de chlore ou de fluor,
T a une des significations indiquées pour X₁ ou représente un groupe alcoxy en C₁ à C₄, un groupe phénoxy éventuellement substitué par un groupe alkyle en C₁ à C₄, alcoxy en C₁ à C₄, un atome d'halogène, un groupe hydroxy, carboxy ou sulfo, un groupe alkylthio en C₁ à C₄ ou un groupe phénylthio éventuellement substitué par un groupe alkyle en C₁ à C₄, alcoxy en C₁ à C₄, un atome d'halogène, un groupe hydroxy, carboxy ou sulfo ou un groupe pipéridino, pirérazino ou morpholino ou indépendamment un groupe U-(B₂)ᵣ-(W₁)ₛ-(B₁)ₜ-W-, dans lequel U, B₁, B₂, W et W₁ ont respectivement la signification indiquée ci-dessus, et
r, s et t représentent indépendamment les uns des autres le nombre 0 ou 1, dans lequel s représente 0 lorsque t est 0,
à la condition que les composés de formule (1) présentent au moins un groupe sulfo ou sulfato et au moins un groupe clivable par un alcalin.

2. Composé selon la revendication 1, caractérisé en ce que, r et s représentent respectivement le nombre 0.

3. Composé selon la revendication 1 ou 2, caractérisé en ce que, B représente un groupe alkylène en C₂ à C₆ non substitué ou substitué par un groupe hydroxy, sulfo ou sulfato, 1,3- ou 1,4-phénylène non substitué ou substitué par un groupe sulfo, carboxy, un atome de chlore, un groupe méthyle ou méthoxy, naphtylène substitué par 1 ou 2 groupes sulfo ou un groupe de formule 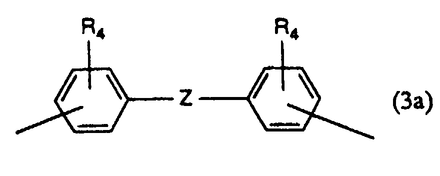 ou 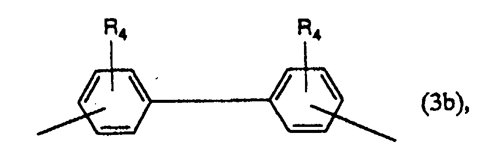 dans lesquelles Z représente -NHCONH-, -O-, -NH-, -CH=CH- ou -CH₂- et R₄ représente un atome d'hydrogène ou un groupe sulfo.

4. Composé selon l'une des revendications 1 à 3, caractérisé en ce que, B représente un groupe 4-sulfophénylène-1,3, 3-sulfophénylène-1,4, 3,6-disulfophénylène-1,4 ou 4,6-disulfophénylène-1,3.

5. Composé selon une des revendications 1 à 4, caractérisé en ce que B₁ est un groupe alkylène en C₁ à C₆ à chaîne linéaire ou ramifiée.

6. Composé selon l'une des revendications 1 à 5, caractérisé en ce que, U représente
(i) un groupe de formule 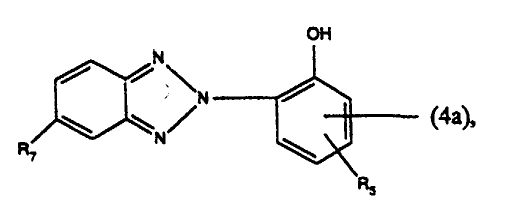 dans laquelle R₅ représente un atome d'hydrogène, un groupe alkyle en C₁ à C₄ ou sulfo et R₇ représente un atome d'hydrogène, un groupe sulfo ou carboxy, et dans laquelle un des groupes R₅ et R₇ est différent d'un atome d'hydrogène;
(ii) un groupe de formule 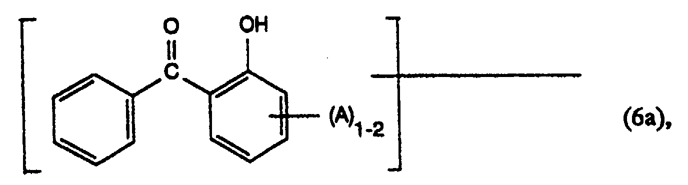 dans laquelle (A)₁₋₂ représente 1 ou 2 groupes identiques ou différents choisis parmi le groupe alcoxy en C₁ à C₁₂ et sulfo
(iii) un groupe de formule 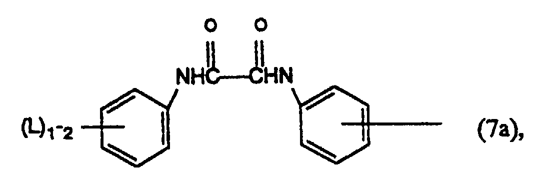 dans laquelle (L)₁₋₂ représente 1 ou 2 groupes L choisis parmi le groupe sulfo, alkyle en C₁ à C₄ et alcoxy en C₁ à C₁₂; ou
(iv) un groupe acide benzoïque non substitué ou substitué par un groupe hydroxy ou alkyle en C₁ à C₄ ou son ester phénylique, alkyle en en C₁ à C₈-phénylique ou alkylique en C₁ à C₁₈,

7. Composé selon l'une des revendications 1 à 6, caractérisé en ce que, R et R' représentent respectivement indépendamment l'un de l'autre un atome d'hydrogène ou un groupe alkyle en C₁ à C₄ et X₁ et X₂ représentent respectivement un atome de chlore ou de fluor.

8. Composé selon l'une des revendications 1 à 7, caractérisé en ce que, W représente un groupe -NR₂- et R₂ représente un atome d'hydrogène ou un groupe alkyle en C₁ à C₄.

9. Composé selon l'une des revendications 1 à 8, caractérisé en ce que, T représente un groupe -NH₂-, N-Mono- ou N,N-Di-alkylamino en C₁ à C₄ non substitué ou substitué dans la partie alkyle par un groupe hydroxy, carboxy, sulfo, sulfato ou alcoxy en C₁ à C₄, cyclohexylamino, phénylamino non substitué ou substitué dans la partie phényle par un groupe alkyle en C₁ à C₄, alcoxy en C₁ à C₄, carboxy, sulfo, un atome d'halogène ou par un groupe de formule
-SO₂-Y (8a),
-CONH-(CH₂)ₚ-SO₂-Y (8b),
dans lesquelles Y représente un groupe vinyle ou un groupe -CH₂-CH₂-G, G est un groupe partant et p un nombre entier de 1 à 6, phénylamino substitué, N-alkyle en C₁ à C₄-N-phénylamino, morpholino, alcoxy en C₁ à C₄ ou un groupe -W-(B₁)ₜ-(W₁)ₛ-(B₂)ᵣ-U.

10. Composé selon l'une des revendications 1 à 9, caractérisé en ce que, T représente un groupe amino, méthylamino, éthylamino, carboxyméthylamino, β-hydroxyéthylamino, β-sulfoéthylamino, N,N-di-β-hydroxyéthylamino, cyclohexylamino, o-, m- ou p-méthylphénylamino, o-, m- ou p-méthoxyphénylamino, o-, m- ou p-sulfophénylamino, 2,4- ou 2,5-disulfophénylamino, o-carboxyphénylamino, N-éthyl-N-phénylamino, N-méthyl-N-phénylamino ou phénylamino qui est substitué par un groupe des formules
-SO₂-Y (8a)
ou
-CONH-(CH₂)ₚ-SO₂-Y (8b),
dans lesquelles Y représente un groupe vinyle ou un groupe -CH₂-CH₂-G, G est un groupe partant et p un nombre entier de 1 à 6.

11. Composé de formule (1) selon l'une des revendications 1 à 9, caractérisé en ce que, T représente un groupe de formule -W-(B₁)ₜ-U et r et s représentent respectivement le nombre 0.

12. Composé selon la revendication 1 de formule 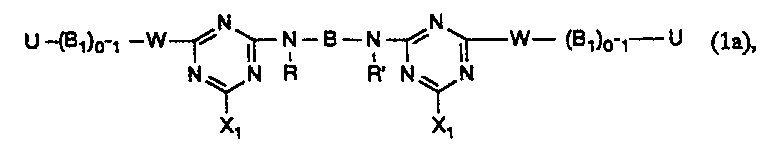 dans laquelle B représente un groupe 4-sulfophénylène-1,3, 3-sulfophénylène-1,4, 3,6-disulfophénylène-1,4 ou 4,6-disulfophénylène-1,3, B₁ représente un groupe alkylène en C₁ à C₆ à chaîne linéaire ou ramifiée, R et R' représentent indépendamment l'un de l'autre respectivement un atome d'hydrogène, un groupe méthyle ou éthyle, W représente le groupe -NH-, X₁ et X₂ représentent respectivement un atome de chlore ou de fluor, et U
(i) un groupe de formule 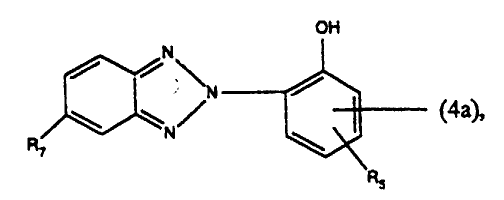 dans laquelle R₅ représente un atome d'hydrogène, un groupe alkyle en C₁ à C₄ ou sulfo et R₇ représente un atome d'hydrogène, un groupe sulfo ou carboxy, et dans laquelle un des groupes R₅ et R₇ est différent d'un atome d'hydrogène;
(ii) un groupe de formule 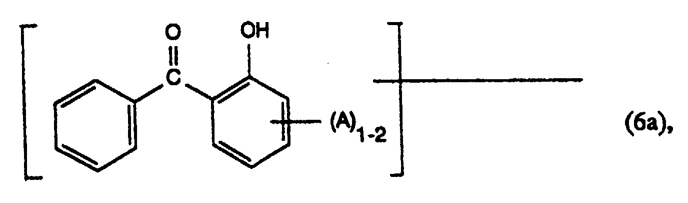 dans laquelle (A)₁₋₂ représente 1 ou 2 groupes identiques ou différents choisis parmi le groupe alcoxy en C₁ à C₁₂ et sulfo
(iii) un groupe de formule 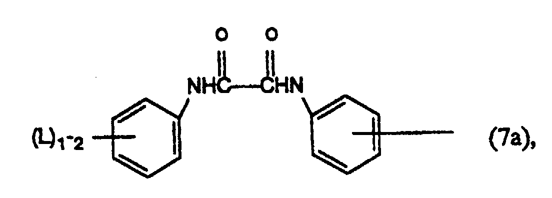 dans laquelle (L)₁₋₂ représente 1 ou 2 groupes L choisis parmi le groupe sulfo, alkyle en C₁ à C₄ et alcoxy en C₁ à C₁₂; ou
(iv) un groupe acide benzoïque non substitué ou substitué par un groupe hydroxy ou alkyle en C₁ à C₄ ou son ester phénylique, alkyle en en C₁ à C₈-phénylique ou alkylique en C₁ à C₁₈.

13. Composé de formule (1a) selon la revendication 12, caractérisé en ce que, U est un groupe de formule (7a).

14. Procédé de préparation de composés de formule (1) selon la revendication 1, caractérisé en ce que, l'on met à réagir ensemble un composé de formule
U-(B₂)ᵣ-(W₁)ₛ-(B₁)ₜ-W-H (8)
un composé de formule
H-RN-B-NR'-H (9),
un composé de formule 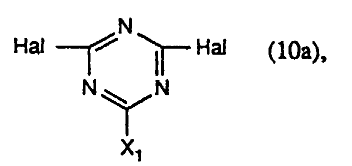 un composé de formule 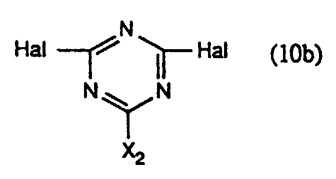 et
éventuellement un composé de formule
T*-H (11),
dans lesquelles U, B, B₁, B₂, W, W₁, R, R', X₁, X₂, r, s et t ont respectivement la signification indiquée à la revendication 1, Hal représente un atome d'halogène, de préférence le fluor ou le chlore et T* a la signification précédemment indiquée pour T à l'exception d'un atome d'halogène, dans l'ordre préféré.

15. Procédé destiné à l'augmentation du facteur de protection solaire de matériaux de fibres textiles, caractérisé en ce que, l'on dépose un ou plusieurs composés de formule (1) selon la revendication 1 en solution aqueuse ou aqueuse-organique sur les matériaux des fibres et en ce qu'on le fixe ensuite.

16. Utilisation de composés de formule (1) selon la revendication 1 pour l'augmentation du facteur de protection contre la lumière de matériaux de fibres textiles non colorés, colorés ou imprimés.

17. Utilisation de composés de formule (1) selon la revendication 1 pour la stabilisation photochimique de matériaux de fibres textiles non colorés, colorés ou imprimés.
